(19) Europäisches Patentamt
European Patent Office
Office européen des brevets

(11) EP 2 855 427 B1

(12) **FASCICULE DE BREVET EUROPEEN**

(45) Date de publication et mention de la délivrance du brevet:
**19.04.2017 Bulletin 2017/16**

(21) Numéro de dépôt: **13739803.8**

(22) Date de dépôt: **04.06.2013**

(51) Int Cl.:
***C07C 257/12*** *(2006.01)* ***C07C 235/02*** *(2006.01)*
***C07C 235/06*** *(2006.01)* ***C07D 239/70*** *(2006.01)*
***C07D 239/74*** *(2006.01)* ***C07D 239/88*** *(2006.01)*
***C07D 263/56*** *(2006.01)* ***C07D 471/04*** *(2006.01)*
***C07D 473/00*** *(2006.01)*

(86) Numéro de dépôt international:
**PCT/IB2013/054601**

(87) Numéro de publication internationale:
**WO 2013/182993 (12.12.2013 Gazette 2013/50)**

(54) **PROCÉDÉ DE PRÉPARATION DE COMPOSÉS AZOTÉS**

VERFAHREN ZUR HERSTELLUNG VON STICKSTOFFVERBINDUNGEN

METHOD FOR PREPARING NITROGEN COMPOUNDS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.06.2012 FR 1255238**

(43) Date de publication de la demande:
**08.04.2015 Bulletin 2015/15**

(73) Titulaire: **Commissariat à l'Énergie Atomique et aux Énergies Alternatives**
**75015 Paris (FR)**

(72) Inventeurs:
- **CANTAT, Thibault**
  **F-92130 Issy Les Moulineaux (FR)**
- **GOMES, Christophe**
  **F-92160 Antony (FR)**
- **JACQUET, Olivier**
  **F-91400 Orsay (FR)**

(74) Mandataire: **Gevers & Orès**
**41 avenue de Friedland**
**75008 Paris (FR)**

(56) Documents cités:
**EP-A1- 0 652 202**

- **ALINEZHAD, HESHMATOLLAH ET AL: "Synthesis of Benzimidazole Derivatives Using Heterogeneous ZnO Nanoparticles", SYNTHETIC COMMUNICATIONS, vol. 42, no. 1, janvier 2012 (2012-01), pages 102-108, XP002686063, ISSN: 0039-7911**
- **WEITH, W.: "Methenyldiphenyldiamine", CHEMISCHE BERICHTE, vol. 9, 1876, pages 454-463, XP002686064,**
- **ANGELA DIBENEDETTO ET AL: "Reaction of silylalkylmono- and silylalkyldi-amines with carbon dioxide: evidence of formation of inter- and intra-molecular ammonium carbamates and their conversion into organic carbamates of industrial interest under carbon dioxide catalysis", GREEN CHEMISTRY, vol. 4, no. 5, 29 juillet 2002 (2002-07-29), pages 439-443, XP055009762, ISSN: 1463-9262, DOI: 10.1039/b205319p**
- **TOSHIYASU SAKAKURA ET AL: "Transformation of carbon dioxide", CHEMICAL REVIEWS, vol. 107, no. 6, 1 juin 2007 (2007-06-01), pages 2365-2387, XP002657706, AMERICAN CHEMICAL SOCIETY, US ISSN: 0009-2665, DOI: 10.1021/CR068357U [extrait le 2007-06-13] cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 855 427 B1

## Description

La présente invention concerne un procédé de préparation de composés azotés utilisant le dioxyde de carbone et l'utilisation de ce procédé dans la fabrication de vitamines, de produits pharmaceutiques, de colles, de fibres acryliques et cuirs synthétiques, de pesticides, d'herbicides, d'antifongiques, et d'engrais.

La présente invention concerne, en outre, un procédé de préparation de composés azotés marqués utilisant le dioxyde de carbone et leurs utilisations.

Elle concerne également un procédé de fabrication de vitamines, de produits pharmaceutiques, de colles, de fibres acryliques, de cuirs synthétiques, de pesticides, d'herbicides, d'antifongiques, et d'engrais, comprenant une étape de préparation de composés azotés par le procédé selon l'invention.

Les ressources carbonées fossiles (pétrole, charbon, gaz) couvrent 85% des besoins énergétiques mondiaux et servent de matières premières pour 95% des consommables chimiques organiques (plastiques, engrais, pesticides, etc.). L'amenuisement des ressources pétrolières et l'accumulation du $CO_2$ résultant de leur utilisation posent donc un problème écologique, énergétique et de disponibilité en matières premières pour l'industrie chimique. Dans ce contexte, il convient de proposer de nouvelles voies de synthèse des consommables chimiques, de manière à construire une industrie durable basée sur l'utilisation de ressources carbonées renouvelables.

Contourner la pétrochimie et valoriser au maximum son déchet carboné, le $CO_2$, pour produire des molécules organiques comme les polymères, les engrais, les textiles synthétiques, etc., représente donc un enjeu scientifique de premier plan. En effet, le recyclage du $CO_2$ présente le double avantage d'économiser des ressources carbonées fossiles (charbon, hydrocarbures, etc.) habituellement utilisées pour synthétiser les molécules organiques, tout en évitant une augmentation des émissions de ce gaz à effet de serre.

Cependant, la mise au point de réactions chimiques permettant de transformer le $CO_2$, notamment en substituant toutes les liaisons C-O du $CO_2$ par de nouvelles liaisons C-H, C-C, C-N, C-S, etc., présente des difficultés techniques.

En effet, compte tenu de la grande stabilité thermodynamique du dioxyde de carbone, sa transformation nécessite une source énergétique extérieure de manière à favoriser le bilan thermodynamique de la transformation chimique et l'utilisation de catalyseur pour accélérer les réactions.

Alinezhad, Heshmatollat et al. (Synthetic Communications (2012), 42 (1), 102-108) et Weith W. (Chemische Berichte (1876), 9, 454-458) décrivent des procédés de production de composés azotés à partir d'une amine et de l'acide formique.

Alors que le $CO_2$ est souvent proposé comme source de carbone pour sa valorisation en intermédiaires pour la chimie (Sakakura, T.; Choi, J. C.; Yasuda, H. Chem Rev 2007, 107, 2365), la seule réaction au cours de laquelle toutes les liaisons C-O sont rompues et de nouvelles liaisons sont formées est la réduction de $CO_2$ en méthane (A. J. Morris, G. J. Meyer, E. Fujita, Acc. Chem. Res. 2009, 42, 1983 ; A. Berkefeld, W. E. Piers, M. Parvez, J. Am. Chem. Soc. 2010, 132, 10660). Tous les autres procédés connus conduisent à des molécules présentant toujours des liaisons C-O et/ou C=O, issues du $CO_2$, avec une reconstruction partielle de la valence du carbone (comme dans le méthanol, l'urée, les formamides, etc.).

Comme exemple de conversion du $CO_2$ vers de nouveaux consommables chimiques en utilisant un partenaire chimique réactif (haut en énergie) pour favoriser le bilan thermodynamique de la transformation chimique de $CO_2$, la synthèse industrielle de l'urée obtenue par condensation d'ammoniac sur $CO_2$, peut être citée (Sakakura, T.; Choi, J. C.; Yasuda, H. Chem Rev 2007, 107, 2365). Cette synthèse est indiquée dans l'équation 1 ci-dessous.

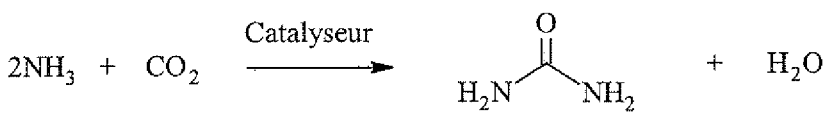


(équation 1)

Selon le même principe, la synthèse de polycarbonates par copolymérisation $CO_2$/époxydes est en voie d'industrialisation comme indiqué dans l'équation 2 ci-dessous (Panorama des voies de valorisation du $CO_2$, ADEME, Juin 2010, http://www2.ademe.fr/servlet/getDoc?cid=96&m=3&id=72052&p1=30&ref=12441).

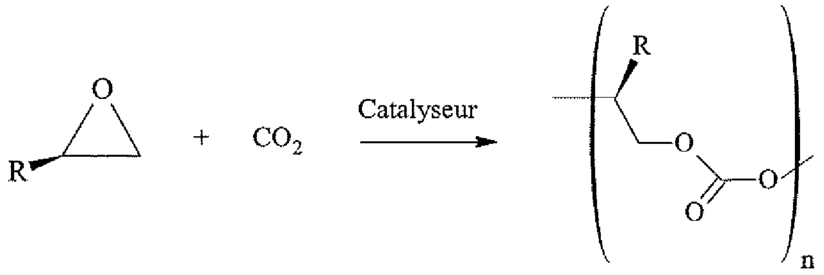


(équation 2)

Dans ces deux synthèses (équations 1 et 2), il n'y a aucune réduction formelle du centre carboné du $CO_2$. Une liaison C=O de chaque $CO_2$ transformé est conservée dans la structure finale.

Toujours dans le but d'obtenir de nouveaux consommables chimiques, la stratégie envisagée peut consister à modifier entièrement la sphère de coordination du $CO_2$ en remplaçant toutes les liaisons C-O du $CO_2$ par de nouvelles liaisons du type C-N, C-H, C-C, C-S et/ou C-O.

A ce jour, la réduction du $CO_2$ en méthane est le seul procédé catalytique connu pour convertir le $CO_2$ en une molécule dans laquelle les quatre liaisons C-O du $CO_2$ ont été remplacées par quatre liaisons C-H.

Cette stratégie peut également s'appliquer pour convertir le $CO_2$ en composés azotés choisis parmi, par exemple, les amidines, les hétérocycles azotés, etc. qui sont une classe de composés chimiques importants dans l'industrie chimique où ils sont couramment utilisés comme réactifs, médicaments, pesticides, herbicides, antifongiques, etc.

Parmi les amidines, les formamidines de formule générale $R^1NCHNR^5R^6$, sont généralement synthétisés par condensation des amines primaires avec des formamides en présence d'un agent déshydratant fort tel que le chlorure de thionyle ($SOCl_2$), le trichlorophosphate, ou l'anhydride trifluoroacétique (M. Gall, J. M. McCall, R. E. TenBrink, P. F. VonVoigtlander, J. S. Mohrland, Journal of Medicinal Chemistry, 1988, 31, 1816-1820 ; S. Enthaler, K. Schrôder, S. Inoue,B. Eckhardt, K. Junge, M. Beller, M. Drieß, European Journal of Organic Chemistry, 2010 , # 25, p. 4893 - 4901 ; K.-M. Cheng, Y.-Y. Huang, J.-J. Huang, K. Kaneko, M. Kimura, H. Takayama, S.-H. Juang, F. F. Wong Bioorganic and Medicinal Chemistry Letters, 2010 , vol.20, # 22, p. 6781-6784 ; A. Mekhalfia, R. Mutter, W. Heal, B. Chen, Tetrahedron, 2006, vol.62, # 24, p. 5617 - 5625). Ces voies de synthèse requièrent généralement un chauffage modéré (60°C) à fort (180°C) et nécessitent l'emploi de réactifs toxiques ($SOCl_2$, $POCl_3$, etc.).

Une voie alternative repose sur la synthèse des formamidines par réaction entre une amine primaire et un trialkylorthoformiate qui est généralement $(EtO)_3CH$ ou $(MeO)_3CH$ (Khaksar, Samad; Vahdat, Seyed Mohammad; Heydari, Akbar; Tajbakhsh, Mahmood, Journal of Fluorine Chemistry, 2010, vol.131; nb. 12, p. 1377 - 1381.). Appliquées aux diamines, cette réaction est utilisée pour la synthèse de hétérocycles azotés comme les benzimidazoles, les quinazolinones, les 3,4-dihydroquinazolines, etc.

Afin d'utiliser le $CO_2$ comme source de carbone pour synthétiser de composés chimiques azotés choisis parmi, par exemple, les amidines, les hétérocycles azotés, etc., dans lesquels toutes les liaisons C-O du $CO_2$ de départ sont remplacées par de nouvelles liaisons du type C-N, C-H et/ou C-O, il faut résoudre un défi technique qui consiste à coupler la fonctionnalisation du $CO_2$ à une étape de réduction chimique et une étape de désoxygénation. Pour maximiser le rendement énergétique d'une telle transformation, il est nécessaire de développer des réactions avec un nombre limité d'étapes (idéalement une seule) et catalysées, pour éviter les pertes énergétiques d'ordre cinétique.

Il existe donc un réel besoin d'un procédé pour préparer les composés azotés choisis parmi, par exemple, les amidines, les hétérocycles azotés, etc., par transformation du $CO_2$, notamment en remplaçant toutes les liaisons C-O du $CO_2$ de départ par de nouvelles liaisons du type C-N, C-H et/ou C-O, palliant les inconvénients de l'art antérieur, ledit procédé permettant de coupler la fonctionnalisation du dioxyde de carbone à une étape de réduction chimique et à une étape de désoxygénation.

En particulier, il existe un réel besoin d'un procédé qui permette d'obtenir en une seule étape et avec une très bonne sélectivité les composés azotés, à partir du $CO_2$ et d'amines, en conditions catalytiques, en présence d'un composé assurant la réduction de $CO_2$ et d'un composé assurant la fonctionnalisation du $CO_2$.

Par ailleurs, les composés azotés marqués choisis parmi, par exemple, les amidines marquées, les hétérocycles azotés marqués, etc., incorporant des radioisotopes et/ou isotopes stables, présentent un intérêt particulier dans de nombreux domaines comme, par exemple, dans les sciences du vivant (étude/élucidation de mécanismes enzymatiques, de mécanismes biosynthétiques, en biochimie, etc.), les sciences de l'environnement (traçage de déchets, etc.), la recherche (étude/élucidation de mécanismes réactionnels) ou encore la recherche et le développement de nouveaux produits pharmaceutiques et thérapeutiques. Ainsi, développer une synthèse pour la préparation de composés azotés marqués répondant aux exigences indiquées ci-dessus, peut répondre à un besoin réel.

Il existe, ainsi, un réel besoin de disposer d'un procédé qui permette d'obtenir, en une seule étape et avec une très bonne sélectivité, les composés azotés marqués choisis parmi, par exemple, les amidines marqués, les hétérocycles azotés marqués, etc., incorporant des radioisotopes et/ou isotopes stables, à partir du $CO_2$ et d'amines, en conditions

catalytiques, en présence d'un composé assurant la réduction de $CO_2$ et d'un composé assurant la fonctionnalisation du $CO_2$, l'un au moins des partenaires réactionnels choisi parmi le $CO_2$, les amines, le composé assurant la réduction du $CO_2$ et le composé assurant la fonctionnalisation du $CO_2$ étant marqué.

La présente invention a précisément pour but de répondre à ces besoins en fournissant un procédé de préparation de composés azotés de formule (I) :

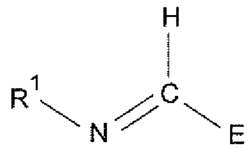

(I)

dans laquelle

- $R^1$ représente un atome d'hydrogène, un groupe alkyle, un groupe alcényle, un groupe alcynyle, un groupe aryle, un groupe hétéroaryle, un hétérocycle, un groupe carbonyle (-CO-), un groupe silylé, un groupe siloxy, un groupe amino, lesdits groupes alkyle, alcényle, alcynyle, aryle, hétéroaryle, hétérocycle, silylé, siloxy et amino étant éventuellement substitués,
- $R^1$ comporte éventuellement un H, C, N, O, F, Si et/ou S tels que définis ci-dessous ;
- H représente un atome d'hydrogène ($^1H$), le deutérium ($^2H$) ou le tritium ($^3H$) ;
- C représente un atome de carbone ($^{12}C$), un isotope $^{11}C$, $^{13}C$ ou $^{14}C$ ;
- N représente un atome d'azote ($^{14}N$), un isotope $^{15}N$ ;
- O représente un atome d'azote ($^{16}O$), un isotope $^{18}O$;
- F représente un atome d'azote ($^{19}F$), un isotope $^{18}F$ ;
- Si représente un atome de silicium ($^{28}Si$), un isotope $^{29}Si$ ou $^{30}Si$ ;
- S représente un atome de soufre ($^{32}S$), un isotope $^{33}S$, $^{34}S$ ou $^{36}S$ ;
- E représente un groupe $R^5R^6N$- ou $R^7O$- avec

  - $R^5$, $R^6$ et $R^7$ représentant, indépendamment l'un de l'autre, un atome d'hydrogène, un groupe alkyle, un groupe alcényle, un groupe alcynyle, un groupe aryle, un groupe hétéroaryle, un hétérocycle, un groupe (-(CO)-), un groupe silylé, un groupe siloxy, un groupe amino, lesdits groupes alkyle, alcényle, alcynyle, aryle, hétéroaryle, hétérocycle, silylé, siloxy et amino étant éventuellement substitués, ou
  - $R^5$ et $R^6$, pris ensemble avec l'atome d'azote auquel ils sont liés forment un hétérocycle éventuellement substitué, ou
  - $R^1$ et $R^5$, pris ensemble avec les deux atomes d'azote auxquels ils sont respectivement liés forment un hétérocycle éventuellement substitué, ou
  - $R^1$ et $R^7$, pris ensemble avec l'atome d'azote et l'atome d'oxygène auxquels ils sont respectivement liés forment un hétérocycle éventuellement substitué,
  - $R^5$, $R^6$ et $R^7$ comportent éventuellement un H, C, N, O, F, Si et/ou S tels que définis ci-dessus,
  - $R^1$, N et O étant tels que définis ci-dessus ;

caractérisé en ce que l'on fait réagir une amine de formule (II) :

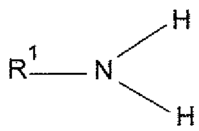

(II)

dans laquelle $R^1$ et N sont tels que définis ci-dessus ; avec du $CO_2$ dans lequel C et O sont tels que définis ci-dessus ; et en présence
d'un catalyseur,
d'un composé silane de formule (III) :

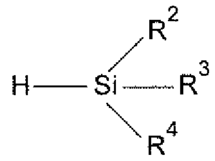

(III)

dans laquelle

- H est tel que défini ci-dessus,
- $R^2$, $R^3$ et $R^4$ représentent, indépendamment l'un de l'autre, un atome d'hydrogène, un atome d'halogène, un groupe hydroxyle, un groupe alkyle, un groupe alcényle, un groupe alcynyle, un groupe alkoxy, un groupe silylé, un groupe siloxy, un groupe aryle, un groupe amino, lesdits groupes alkyle, alcényle, alcynyle, alkoxy, silylé, siloxy, aryle et amino étant éventuellement substitués, ou
- $R^4$ est tel que défini ci-dessus et $R^2$ et $R^3$, pris ensemble avec l'atome de silicium auquel ils sont liés forment un hétérocycle silylé éventuellement substitué, et

d'un agent nucléophile de formule (IV) :

E-H (IV)

dans laquelle E est tel que défini ci-dessus et H est un atome d'hydrogène.

Le procédé de l'invention a l'avantage de permettre de convertir le $CO_2$ éventuellement marqué, en composés azotés éventuellement marqués, avec un grand choix d'amines de formule (II) (amines primaires, secondaires, aromatiques, aliphatiques, insaturés, etc.) éventuellement marquées. Dans ce procédé, les amines de formule (II) et l'agent nucléophile de formule (IV) servent essentiellement à fonctionnaliser le $CO_2$, et les composés silanes de formule (III) assurent la réduction de $CO_2$, en conditions catalytiques.

Le procédé de l'invention permet de remplacer, en une seule étape, les deux atomes d'oxygène du $CO_2$ par des atomes d'hydrogène, d'azote et/ou d'oxygène provenant des autres réactifs (ou partenaires réactionnels) et de conduire à des composés azotés de formule (I) avec un bon rendement (rendement pouvant aller jusqu'à 100%).

Dans le cadre de la présente invention, le rendement est calculé par rapport à la quantité d'amine de formule (II) introduite initialement, sur la base de la quantité de composé azoté de formule (I) isolé :

$$\text{Rendement} = n(\text{composé azoté}) / (n(\text{composé azoté}) + n(\text{amine}))$$

n étant la quantité de matière

Comme déjà indiqué, le procédé de l'invention permet d'obtenir les composés azotés de formule (I) en « une seule étape ». Autrement dit, contrairement aux procédés dans lesquels les amines de départ sont soumises a des réactions chimiques successives avec ajout successif (un à la fois) des autres réactifs, avec ou sans séparation des produits intermédiaires, le procédé de l'invention se fait en « une seule étape » au cours de laquelle l'ensemble des réactifs se trouvent simultanément dans le milieu réactionnel. Ainsi, sur le plan industriel le procédé de l'invention présente un grand intérêt car il permet de gagner en temps, en coût de production et en rendement global.

Dans le cadre de la présente invention, les composés azotés de formule (I) obtenus par le procédé de l'invention peuvent être tout composé comportant un motif -N=CH-N ou -N=CH-O, qui soit acyclique ou mono- ou polycyclique et qui comporte éventuellement une ou plusieurs insaturations (double ou triple liaison) en plus de la double liaison carbone azote du motif -N=CH-N ou -N=CH-O, ledit composé pouvant être éventuellement substitué. A titre d'exemple de composés azotés de formule (I), on peut citer les composés formamidines et les hétérocycles azotés choisis parmi les benzimidazoles, benzoxazoles, quinazolinones ou 3,4-dihydroquinazolines, par exemple.

Par formamidine, on entend une classe de composés organiques cycliques ou acycliques comportant un motif -N=CH-N. Les formamidines sont des homologues de formamides dans lequel la liaison C=O est remplacée par un C=N et sont une sous-classe des amidines de formule -N=CR-N pour laquelle R est un atome d'hydrogène.

Au sens de l'invention, les hétérocycles azotés désignent un substituant mono- ou polycyclique, comportant de 5 à 20 membres dont au moins 2 atomes de carbone, et au moins un atome d'azote et éventuellement au moins un autre hétéroatome choisi parmi l'oxygène ou le soufre. Les hétérocycles azotés peuvent comporter une double liaison carbone azote (N=C), et peuvent encore comprendre une ou plusieurs autres insaturations. Ils peuvent également être aromatiques. A titre d'hétérocycles azotés, on peut citer, imidazoles, benzimidazoles, triazoles, oxazoles, benzoxazoles,

pyrimidines, triazines, quinazolines, les 3,4-dihydroquinazolines, quinazolinones, diazines. L'hétérocycle azoté peut être éventuellement substitué par un ou plusieurs groupes hydroxyle ; un ou plusieurs groupes alkoxy ; un ou plusieurs groupes siloxy ; un ou plusieurs groupes thiol ; un ou plusieurs atomes d'halogène choisis parmi les atomes de fluor, chlore, brome et iode ; un ou plusieurs groupes amino ; un ou plusieurs groupes amido ; un ou plusieurs groupes nitro ($-NO_2$) ; un ou plusieurs groupes nitrile (-CN) ; un ou plusieurs groupes aryle ; un ou plusieurs groupes alkyle ; avec les groupes alkyle, alkoxy, thiol, aryle, amido et amino tels que définis dans le cadre de la présente invention.

On entend par « alkyle », au sens de la présente invention, un radical carboné linéaire, ramifié ou cyclique, saturé ou insaturé, éventuellement substitué, comprenant 1 à 12 atomes de carbone. A titre d'alkyle saturé, linéaire ou ramifié, on peut citer par exemple les radicaux méthyle, éthyle, propyle, butyle, pentyle, hexyle, heptyle, octyle, nonyle, décyle, undécyle, dodécanyle et leurs isomères ramifiés. Comme alkyle cylique, on peut citer les radicaux cyclopropyle, cyclobutyle, cyclopentyle, cyclohexyle, bicylco[2,1,1]hexyle, bicyclo[2,2,1]heptyle. Comme alkyles cycliques insaturés, on peut citer par exemple le cyclopentényle, le cyclohexényle. Les alkyles insaturés appelés également « alcényle » ou « alcynyle » contiennent respectivement au moins une double ou une triple liaison. A ce titre, on peut citer, par exemple, les radicaux éthylényle, propylényle, butényle, pentényle, hexényle, acétylényle, propynyle, butynyle, pentynyle, hexynyle et leurs isomères ramifiés. Le groupe alkyle, au sens de l'invention incluant les groupes alcényle et alcynyle, peut être éventuellement substitué par un ou plusieurs groupes hydroxyle ; un ou plusieurs groupes alkoxy ; un ou plusieurs groupes thiol ; un ou plusieurs atomes d'halogène choisis parmi les atomes de fluor, chlore, brome et iode ; un ou plusieurs groupes amino ; un ou plusieurs groupes amido ; un ou plusieurs groupes nitro ($-NO_2$) ; un ou plusieurs groupes nitrile (-CN) ; un ou plusieurs groupes aryle, avec les groupes alkoxy, thiol, aryle, amino et amido tels que définis dans le cadre de la présente invention.

Le terme « aryle » désigne de manière générale un substituant aromatique cyclique comportant de 6 à 20 atomes de carbone. Dans le cadre de l'invention le groupe aryle peut être mono- ou polycyclique. A titre indicatif, on peut citer les groupes phényle, benzyle, naphtyle, phénanthényle et anthracényle. Le groupe aryle peut être éventuellement substitué par un ou plusieurs groupes hydroxyle ; un ou plusieurs groupes alkoxy ; un ou plusieurs groupes thiol ; un ou plusieurs atomes d'halogène choisis parmi les atomes de fluor, chlore, brome et iode ; un ou plusieurs groupes amino ; un ou plusieurs groupes amido ; un ou plusieurs groupes nitro ($-NO_2$) ; un ou plusieurs groupes nitrile (-CN) ; un ou plusieurs groupes alkyle ; un ou plusieurs groupes aryle ; avec les groupes alkoxy, thiol, alkyle, aryle, amido et amino tels que définis dans le cadre de la présente invention.

Le terme « hétéroaryle » désigne de manière générale un substituant aromatique mono- ou polycyclique comportant de 5 à 20 membres dont au moins 2 atomes de carbone, et au moins un hétéroatome choisi parmi l'azote, l'oxygène ou le soufre. Le groupe hétéroaryle peut être mono- ou poly- cyclique. A titre indicatif, on peut citer les groupes furyle, benzofuranyle, naphtofuranyle, benzopyranyle pyrrolyle, indolyle, isoindolyle, azaindolyle, thiophényle, benzothiophényle, pyridyle, quinolinyle, isoquinolyle, imidazolyle, benzimidazolyle, triazolyle, pyrazolyle, oxazolyle, isoxazolyle, benzoxazolyle, thiazolyle, benzothiazolyle, isothiazolyle, diazinyle, pyridazinyle, pyrimidinyle, pyrazinyle, triazinyle, cinnolinyle, phtalazinyle, quinazolinyle, 1,1-diphénylhydrazinyle, 1,2-diphénylhydrazinyle. Le groupe hétéroaryle peut être éventuellement substitué par un ou plusieurs groupes hydroxyle ; un ou plusieurs groupes alkoxy ; un ou plusieurs groupes thiol ; un ou plusieurs atomes d'halogène choisis parmi les atomes de fluor, chlore, brome et iode ; un ou plusieurs groupes amino ; un ou plusieurs groupes amido ; un ou plusieurs groupes nitro ($-NO_2$) ; un ou plusieurs groupes nitrile (-CN) ; un ou plusieurs groupes aryle ; un ou plusieurs groupes alkyle ; avec les groupes alkyle, alkoxy, thiol, aryle, amido et amino tels que définis dans le cadre de la présente invention.

Le terme « alkoxy » signifie un groupe alkyle, tel que défini ci-dessus, lié par un atome d'oxygène (-O-alkyle).

Le terme « hétérocycle » désigne de manière générale un substituant mono- ou polycyclique, comportant de 5 à 20 membres, saturé ou insaturé, contenant de 1 à 4 hétéroatomes choisis indépendamment l'un de l'autre, parmi l'azote, l'oxygène et le soufre. A titre indicatif, on peut citer les substituants morpholinyle, pipéridinyle, pipérazinyle, pyrrolidinyle, imidazolyle, imidazolidinyle, imidazolinyle, pyrazolidinyle, tétrahydrofuranyle, tétrahydropyranyle, tétrahydropyrimidinyle, triazolyle, pyrazolyle, thianyle, oxazolidinyle, isoxazolidinyle, thiazolidinyle, isothiazolidinyle. L'hétérocycle peut être éventuellement substitué par un ou plusieurs groupes hydroxyle ; un ou plusieurs groupes alkoxy ; un ou plusieurs groupes thiol ; un ou plusieurs groupes aryle ; un ou plusieurs atomes d'halogène choisis parmi les atomes de fluor, chlore, brome et iode ; un ou plusieurs groupes amino ; un ou plusieurs groupes amido ; un ou plusieurs groupes nitro ($-NO_2$) ; un ou plusieurs groupes nitrile (-CN) ; un ou plusieurs groupes alkyle ; avec les groupes alkyle, alkoxy, thiol, aryle, amido et amino tels que définis dans le cadre de la présente invention.

Par atome d'halogène, on entend un atome choisi parmi les atomes de fluor, chlore, brome ou iode.

Par groupe « silylé », on entend un groupe de formule $[-Si(X)_3]$ dans lequel chaque X, indépendamment l'un de l'autre, est choisi parmi un atome d'hydrogène ; un ou plusieurs atomes d'halogène choisis parmi les atomes de fluor, chlore, brome ou iode ; un ou plusieurs groupes alkyle ; un ou plusieurs groupes alkoxy ; un ou plusieurs groupes aryle ; un ou plusieurs groupes siloxy ; un ou plusieurs groupes amino ; un ou plusieurs groupes amido ; avec les groupes alkyle, alkoxy, aryle, amido, amino et siloxy tels que définis dans le cadre de la présente invention. Lorsque l'un au moins des X représente plusieurs groupes siloxy, lesdits groupes siloxy peuvent se répéter plusieurs fois de manière à

conduire à des organosilanes polymériques de formule générale

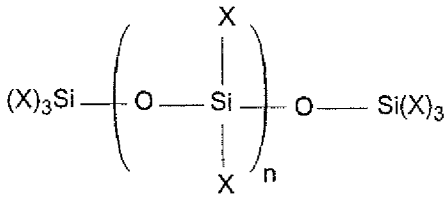

dans laquelle X est tel que défini ci-dessus et n est un nombre entier compris entre 1 et 20000, avantageusement 1000 et 5000. A ce titre on peut citer, par exemple, le polydiméthyl siloxane (PDMS) ou le polyméthylhydrosiloxane (PMHS).

Par groupe « siloxy », on entend un groupe silylé, tel que défini ci-dessus, lié par un atome d'oxygène ($-O-Si(X)_3$).

Au sens de l'invention, par « hétérocycle silylé », on entend un substituant mono- ou polycyclique, comportant de 5 à 15 membres, saturé ou instauré, contenant au moins un atome de silicium et éventuellement au moins un autre hétéroatome choisi parmi l'azote, l'oxygène ou le soufre. Ledit hétérocycle silylé peut être éventuellement substitué par un ou plusieurs groupes hydroxyle ; un ou plusieurs groupes alkyle, un ou plusieurs groupes alkoxy ; un ou plusieurs groupes thiol ; un ou plusieurs atomes d'halogène choisis parmi les atomes de fluor, chlore, brome et iode ; un ou plusieurs groupes aryle ; un ou plusieurs groupes amino ; un ou plusieurs groupes amido ; avec les groupes alkyle, alkoxy, thiol, aryle, amido et amino tels que définis dans le cadre de la présente invention. Parmi les hétérocycles silylés, on peut citer par exemple, le 1-silacyclo-3-pentène ou le 1-méthyl-1,1-dihydrido-2,3,4,5-tétraphényl-1-silacyclopentadiène, suivant les formules ci-dessous.

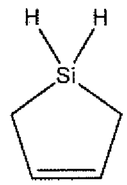

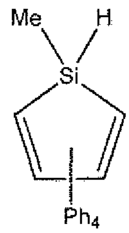

1-silacyclo-3-pentène 1-methyl-1-hydrido-2,3,4,5-tétraphényl-1-silacyclopentadiène

On peut encore citer, par exemple, le méthyl siloxane, le 1-phényl-1-silacyclohexane, le 1-sila-bicyclo[2.2.1] heptane, le 1-méthyl-1-silacyclopentane, le 9,9-dihydro-5-silafluorène répondant aux formules ci-dessous.

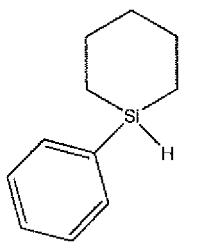

1-phenyl-1-silacyclohexane

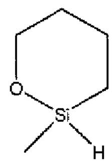

methyl siloxane

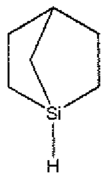

1-sila-bicyclo[2.2.1]heptane

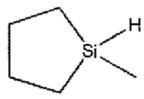

1-methyl-1-silacyclopentane

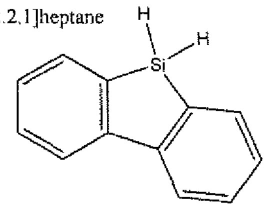

9,9-dihydro-5-silafluorene

Les hétérocycles silylés de l'invention peuvent être disponibles commercialement ou peuvent, le cas échéant, être préparés par les procédés de synthèse connus comme, par exemple, décrits par C.L. Smith et al., Journal of Organometallic Chemistry, 81 (1974), p. 33-40 ; G.D. Homer, Journal of the American Chemical Society, 95:23, (1973), p. 7700-7707 ; L. Spialter et al., Journal of the American Chemical Society, 93:22 (1971), p. 5682-5686 ; R. West, Journal of the American Chemical Society (1954), p. 6015-6017. L'homme du métier sera en mesure de mettre en oeuvre et d'adapter les procédés connus à la synthèse des différents hétérocycles silylés.

Par groupe « amino », on entend un groupe de formule $-NR^8R^9$, dans laquelle : $R^8$ et $R^9$ représentent, indépendamment l'un de l'autre, un atome d'hydrogène, un groupe alkyle, un groupe alcényle, un groupe alcynyle, un groupe aryle, un groupe hétéroaryle, un hétérocycle, un groupe $-CO-R^{10}$, un groupe silylé, un groupe siloxy, avec les groupes alkyle, alcényle, alcynyle, aryle, hétéroaryle, hétérocycle, silylé, siloxy, tels que définis dans le cadre de la présente invention ; ou

$R^8$ et $R^9$, pris ensemble avec l'atome d'azote auquel ils sont liés, forment un hétérocycle éventuellement substitué par un ou plusieurs groupes hydroxyle ; un ou plusieurs groupes alkyle ; un ou plusieurs groupes alkoxy ; un ou plusieurs atomes d'halogène choisis parmi les atomes de fluor, chlore, brome et iode ; un ou plusieurs groupes amino ; un ou plusieurs groupes nitro ($-NO_2$) ; un ou plusieurs groupes nitrile (-CN) ; un ou plusieurs groupes aryle ; avec les groupes alkyle, alkoxy, aryle et amino tels que définis dans le cadre de la présente invention ; et

$R^{10}$ représente, un atome d'hydrogène, un groupe alkyle, un groupe alcényle, un groupe alcynyle, un groupe aryle, un groupe hétéroaryle, un hétérocycle, un groupe silylé, un groupe siloxy, avec les groupes alkyle, alcényle, alcynyle, aryle, hétéroaryle, hétérocycle, silylé, siloxy, tels que définis dans le cadre de la présente invention.

Par groupe « amido », on entend un groupe de formule $-CO-NR^8R^9$, dans laquelle :

$R^8$ et $R^9$ représentent, indépendamment l'un de l'autre, un atome d'hydrogène, un groupe alkyle, un groupe alcényle, un groupe alcynyle, un groupe aryle, un groupe hétéroaryle, un hétérocycle, un groupe $-CO-R^{10}$, un groupe silylé, un groupe siloxy, avec les groupes alkyle, alcényle, alcynyle, aryle, hétéroaryle, hétérocycle, silylé, siloxy, tels que définis dans le cadre de la présente invention ; ou

$R^8$ et $R^9$, pris ensemble avec l'atome d'azote auquel ils sont liés, forment un hétérocycle éventuellement substitué par un ou plusieurs groupes hydroxyle ; un ou plusieurs groupes alkyle ; un ou plusieurs groupes alkoxy ; un ou plusieurs groupes thiol ; un ou plusieurs atomes d'halogène choisis parmi les atomes de fluor, chlore, brome et iode ; un ou plusieurs groupes amino ; un ou plusieurs groupes nitro ($-NO_2$) ; un ou plusieurs groupes nitrile (-CN) ; un ou plusieurs groupes aryle ; avec les groupes alkyle, alkoxy, thiol, aryle et amino tels que définis dans le cadre de la présente invention ; et

$R^{10}$ représente, un atome d'hydrogène, un groupe alkyle, un groupe alcényle, un groupe alcynyle, un groupe aryle, un groupe hétéroaryle, un hétérocycle, un groupe silylé, un groupe siloxy, avec les groupes alkyle, alcényle, alcynyle, aryle, hétéroaryle, hétérocycle, silylé, siloxy, tels que définis dans le cadre de la présente invention.

Par groupe « thiol » on entend un groupe de formule $-SR^{12}$, dans laquelle $R^{12}$ représente, un atome d'hydrogène, un groupe alkyle, un groupe alcényle, un groupe alcynyle, un groupe aryle, un groupe hétéroaryle, un hétérocycle, un groupe silylé, un groupe siloxy, avec les groupes alkyle, alcényle, alcynyle, aryle, hétéroaryle, hétérocycle, silylé, siloxy, tels que définis dans le cadre de la présente invention.

Les substituants, radicaux et groupes définis ci-dessus peuvent comporter, éventuellement, du deutérium ($^2H$), du tritium ($^3H$), du $^{11}C$, du $^{13}C$, du $^{14}C$, du $^{15}N$, du $^{18}O$, du $^{18}F$, du $^{29}Si$, du $^{30}Si$, du $^{33}S$, du $^{34}S$ ou du $^{36}S$.

Lorsque les composés de formule (I), (II), (III) et (IV) comporte au moins un radiomarqueur/radiotraceur ou un isotope, ils peuvent également être désignés par les formules (I'), (II'), (III') et (IV').

Selon une variante préférée de l'invention, dans l'amine de formule (II), $R^1$ représente un atome d'hydrogène, un groupe alkyle, un groupe aryle, un groupe hétéroaryle, un hétérocycle, un groupe amino, lesdits groupes alkyle, amino, aryle, hétérocycle et hétéroaryle étant éventuellement substitués.

Selon une autre variante préférée, dans l'amine de formule (II), $R^1$ représente un atome d'hydrogène ; un groupe alkyle choisi parmi les groupes méthyle, éthyle, propyle, butyle, pentyle, hexyle, heptyle, ou leurs isomères ramifiés, cyclohexyle ; un groupe aryle choisi parmi le benzyle, le phényle ou le naphtyle ; un groupe hétéroaryle choisi parmi le diazinyle, le pyridazinyle, le pyrimidinyle, le pyrazinyle, l'imidazolyle ou le benzimidazolyle ; lesdits groupes alkyle, aryle et hétéroaryle étant éventuellement substitués.

Selon une autre variante préférée de l'invention, dans le composé silane de formule (III), $R^2$, $R^3$ et $R^4$ représentent, indépendamment l'un de l'autre, un atome d'hydrogène, un groupe alkyle, un groupe alkoxy, un groupe aryle, un groupe silylé, un groupe siloxy, lesdits groupes alkyle, alkoxy, silylé, siloxy et aryle étant éventuellement substitués.

De préférence, dans le composé silane de formule (III), $R^2$, $R^3$ et $R^4$ représentent indépendamment l'un de l'autre,

- un atome d'hydrogène ;
- un groupe alkyle choisi parmi les groupes méthyle, éthyle, propyle, butyle, pentyle, hexyle, heptyle ou leurs isomères ramifiés ;
- un groupe alkoxy dont le groupe alkyle est choisi parmi les groupes méthyle, éthyle, propyle, butyle, pentyle, hexyle, heptyle ou leurs isomères ramifiés ;
- un groupe aryle choisi parmi les groupes benzyle ou phényle ;
- un groupe silylé de formule $[-Si(X)_3]$ dans lequel chaque X, indépendamment l'un de l'autre, est choisi parmi un atome d'hydrogène ; un ou plusieurs atomes d'halogène choisis parmi les atomes chlore, brome, ou iode ; un ou plusieurs groupes alkyle choisi parmi les groupes méthyle, éthyle, propyle, butyle, pentyle, hexyle, heptyle ou leurs isomères ramifiés ; un ou plusieurs groupes alkoxy dont le groupe alkyle est choisi parmi les groupes méthyle, éthyle, propyle, butyle, pentyle, hexyle, heptyle ou leurs isomères ramifiés ; un ou plusieurs groupes siloxy dont le groupe $-Si(X)_3$ est tel que décrit dans ce mode de réalisation ; plusieurs groupes siloxy se répétant plusieurs fois conduisant à des organosilanes polymériques de formule générale

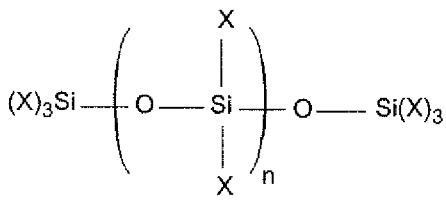

dans laquelle X est tel que défini dans ce mode de réalisation et n est un nombre entier compris entre 1 et 20000, avantageusement entre 1000 et 5000.

Comme déjà indiqué, tout comme l'amine, l'agent nucléophile E-H de formule (IV) sert à fonctionnaliser le $CO_2$.

Selon une variante préférée de l'invention, E représente un groupe $R^5R^6N$- avec

- $R^5$ et $R^6$ représentant, indépendamment l'un de l'autre, un atome d'hydrogène, un groupe alkyle, un groupe aryle, un groupe hétéroaryle, un hétérocycle, un groupe amino, lesdits groupes alkyle, aryle, hétéroaryle, hétérocycle et amino étant éventuellement substitués, ou
- $R^5$ et $R^6$, pris ensemble avec l'atome d'azote auquel ils sont liés forment un hétérocycle éventuellement substitué.

De préférence, E représente un groupe $R^5R^6N$- avec $R^5$ et $R^6$, représentant, indépendamment l'un de l'autre, un atome d'hydrogène; un groupe alkyle choisi parmi les groupes méthyle, éthyle, propyle, butyle, pentyle, hexyle, heptyle, ou leurs isomères ramifiés, cyclohexyle ; un groupe aryle choisi parmi le benzyle, le phényle ou le naphtyle ; un groupe hétéroaryle choisi parmi le diazinyle, le pyridazinyle, le pyrimidinyle, le pyrazinyle, l'imidazolyle ou le benzimidazolyle ; lesdits groupes alkyle, aryle et hétéroaryle étant éventuellement substitués.

L'amine de formule (II) et l'agent nucléophile de formule (IV) sont deux composés distincts qui peuvent être identiques ou non selon que l'on cherche à obtenir un composé azoté symétrique ou asymétrique. Selon une variante particulièrement préféré, l'agent nucléophile E-H de formule (IV) et l'amine de formule (II) sont identiques.

Par catalyseur, au sens de l'invention, on entend tout composé capable de modifier, notamment en augmentant, la vitesse de la réaction chimique à laquelle il participe, et qui est régénéré à la fin de la réaction. Cette définition englobe à la fois les catalyseurs, c'est-à-dire les composés qui exercent leur activité catalytique sans avoir besoin de subir une quelconque modification ou conversion, et les composés (appelés également pré-catalyseurs) qui sont introduits dans

le milieu réactionnel et qui y sont convertis en un catalyseur.

Les catalyseurs peuvent être choisis parmi les catalyseurs organiques ou les catalyseurs métalliques choisis parmi les sels ou complexes métalliques. Les catalyseurs organiques présentent l'avantage de permettre de s'affranchir des problèmes de toxicité généralement observés pour les catalyseurs métalliques ainsi que des problèmes de coûts associés à l'utilisation de métaux précieux. Dans le procédé de l'invention, le catalyseur est, de préférence, organique.

Les catalyseurs organiques sont, en général, des bases organiques, choisies parmi :

- les bases azotées, comme par exemple, les amines secondaires ou tertiaires choisies parmi le triazabicyclodécène (TBD) ; le N-méthyltriazabicyclodécène (MeTBD), le 1,8-diazabicyclo[5.4.0]undéc-7-ène (DBU), la triméthylamine, la triéthylemaine, la pipéridine, la 4-diméthylaminopyridine (DMAP), le 1,4-diazabicyclo[2.2.2]octane (DABCO), la proline, le phénylalanine, un sel de thiazolium, la *N*-diisopropyléthylamine (DIPEA ou DIEA) ;
- les bases phosphorées, comme par exemple, les alkyles et aryle phosphines choisi parmi le triphénylphosphine, le 2,2'-bis(diphénylphosphino)-1,1'-binaphthyle (BINAP), le triisopropylphosphine ; les alkyle et aryle phosphonates choisis parmi le diphénylphosphate, le triphénylphosphate (TPP), le tri(isopropylphényl)phosphate (TIPP), le crésyldiphenyl phosphate (CDP), le tricrésylphosphate (TCP) ; les alkyle et aryle phosphates choisis parmi le di-n-butylphosphate (DBP), le tris-(2-éthylhexyl)-phosphate, le triéthyl phosphate ;
- les bases carbonées pour lesquelles la protonation a lieu sur un atome de carbone, comme par exemple, un carbène N-hétérocyclique comme un carbène issu d'un sel d'imidazolium choisi parmi les sels de 1,3-bis(2,6-diisopropylphenyl)-1H-imidazol-3-ium (carbène A), 1,3-bis(2,6-diisopropylphenyl)-4,5-dihydro-1H-imidazol-3-ium (carbène C), 1,3-bis(2,4,6-triméthylphenyl)-1H-imidazol-3-ium (carbène B), 1,3-bis(2,4,6-triméthylphenyl)-4,5-dihydro-1H-imidazol-3-ium (carbène D), 4,5-dichloro-1,3-bis(2,6-diisopropylphenyl)-1H-imidazol-3-ium (carbène E), 1,3-di-tert-butyl-1H-imidazol-3-ium (carbène F), 1,3-di-tert-butyl-4,5-dihydro-1H-imidazol-3-ium, lesdits sels étant, par exemple, sous la forme de sels de chlorure comme représentés ci-dessous :

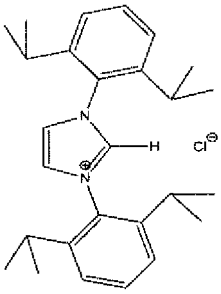
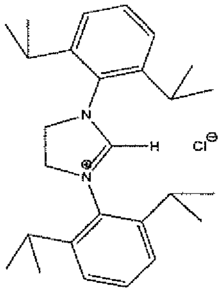
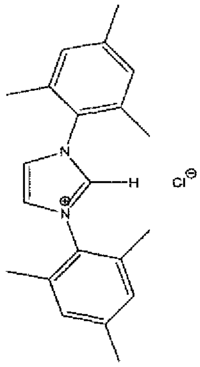
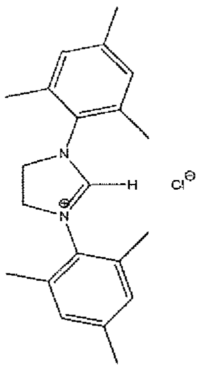
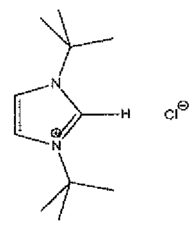
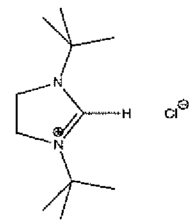
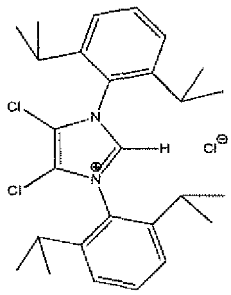

ou

- les bases oxygénées, comme par exemple le peroxyde d'hydrogène ; le peroxyde de benzoyle ; un alcoolate choisi parmi le méthanolate, l'éthanolate, le propanolate, le butanolate, le pentanolate, l'hexanolate, de sodium ou de potassium.

Le catalyseur organique est avantageusement :

- une amine secondaire ou tertiaire choisie parmi le triazabicyclodécène (TBD) ; le N-méthyltriazabicyclodécène (MeTBD), le 1,8-diazabicyclo[5.4.0]undéc-7-ène (DBU), la triméthylamine, la triéthylemaine, la pipéridine, la 4-diméthylaminopyridine (DMAP), le 1,4-diazabicyclo[2.2.2]octane (DABCO), la proline, le phénylalanine, un sel de thiazolium, la *N*-diisopropyléthylamine (DIPEA ou DIEA) ; ou

- un carbène N-hétérocyclique comme un carbène issu d'un sel d'imidazolium choisi parmi les sels de 1,3-bis(2,6-diisopropylphenyl)-1H-imidazol-3-ium (carbène A), 1,3-bis(2,6-diisopropylphenyl)-4,5-dihydro-1H-imidazol-3-ium (carbène C), 1,3-bis(2,4,6-triméthylphenyl)-1H-imidazol-3-ium (carbène B), 1,3-bis(2,4,6-triméthylphenyl)-4,5-dihydro-1H-imidazol-3-ium (carbène D), 4,5-dichloro-1,3-bis(2,6-diisopropylphenyl)-1H-imidazol-3-ium (carbène E), 1,3-di-tert-butyl-1H-imidazol-3-ium (carbène F), 1,3-di-tert-butyl-4,5-dihydro-1H-imidazol-3-ium, lesdits sels étant, par exemple, sous la forme de sels de chlorure comme représentés ci-dessous :

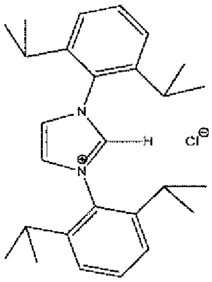
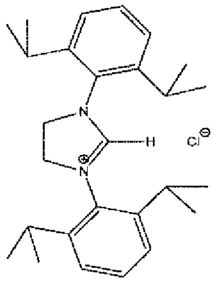
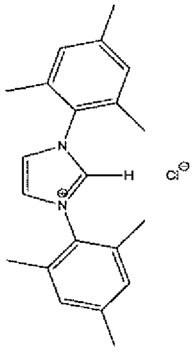
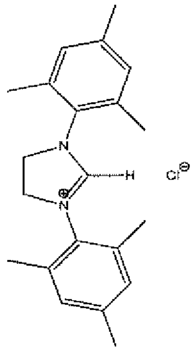

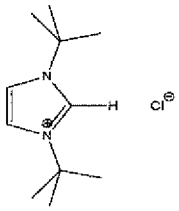
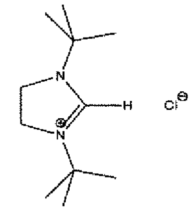
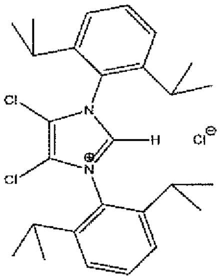

Selon une variante préférée de l'invention, le catalyseur organique est choisi parmi le triazabicyclodécène (TBD), le N-méthyltriazabicyclodécène (MeTBD), le 1,8-diazabicyclo[5.4.0]undéc-7-ène (DBU) ; ou un carbène N-hétérocyclique tel qu'un carbène issu d'un sel d'imidazolium tel que le chlorure de 1,3-bis(2,6-diisopropylphenyl)-1H-imidazol-3-ium (carbène A), le chlorure de 1,3-bis(2,6-diisopropylphenyl)-4,5-dihydro-1H-imidazol-3-ium (carbène C), le chlorure de 1,3-di-tert-butyl-1H-imidazol-3-ium, le chlorure de 1,3-di-tert-butyl-4,5-dihydro-1H-imidazol-3-ium (carbène F), le chlorure de 1,3-bis(2,4,6-triméthylphenyl)-1H-imidazol-3-ium (carbène B), le chlorure de 1,3-bis(2,4,6-triméthylphenyl)-4,5-dihydro-1H-imidazol-3-ium (carbène D), le chlorure de 4,5-dichloro-1,3-bis(2,6-diisopropylphenyl)-1H-imidazol-3-ium (carbène E).

Lorsque le catalyseur est un catalyseur métallique, il peut être choisi parmi les sels ou complexes de :

- métaux choisis parmi le bore, le silicium, l'aluminium, le gallium, l'étain, l'indium ;
- métaux alcalins choisis parmi le sodium, le potassium ;
- métaux alcalinoterreux choisis parmi le magnésium, le calcium ;
- métaux de transition choisis parmi le nickel, le fer, le cobalt, le zinc, le cuivre, le rhodium, le ruthénium, le platine, le palladium, l'iridium ;
- terres rares choisis parmi le lanthane, le cérium, le praséodyme, le néodyme.

A titre d'exemples, le catalyseur métallique, peut être choisi parmi les sels ou complexes suivants :

- $Al(OiPr)_3$, $SnCl_2$, $InBr_3$ en tant que sels ou complexes de métaux ;
- $Na_2CO_3$, $K_2CO_3$, $Cs_2CO_3$ en tant que sels ou complexes de métaux alcalins ;
- $MgSO_4$, $Ca(BH_4)_2$ en tant que sels ou complexes de métaux alcalinoterreux ;
- $Fe(BH_4)_2.6H_2O$, $Fe(BF_4)_2.6H_2O$, $Fe(acac)_3$, CuCl, $Cu(OAc)_2(H_2O)$, $Zn(OAc)_2$, Zn(BDI)Et, $ZnEt_2$, $ZnCl_2$, $ZnSO_4$, en tant que sels ou complexes de métaux de transition ;
- $La(OTf)_3$, $CeCl_3$ en tant que sels ou complexes de terres-rares.

Par complexe métallique il est entendu un composé organométallique ou inorganique de coordination dans lequel un ion métallique est lié à un ligand organique ou inorganique. Un complexe organométallique ou inorganique peut être obtenu par mélange d'un sel métallique avec un ligand, celui-ci se liant au métal par des atomes de phosphore,

de carbone, d'azote, d'oxygène, d'hydrogène ou de silicium, par exemple. Comme ligand organique ou inorganique, on peut citer par exemple, les phosphines (PRR'R") ; les amines (NRR'R") ; les éthers (R-O-R') ; les carbènes ; les thiols (R-S-H) ; les amidines ; les guanidines (RR'N)($R^aR^b$N)C=N-R") ; avec R, R', R", $R^a$ et $R^b$ représentant, indépendamment l'un de l'autre, un atome d'hydrogène, un groupe alkyle, un groupe un groupe aryle, un groupe hétéroaryle, un hétérocycle, avec les groupes alkyle, aryle, hétéroaryle et hétérocycle tels que définis dans le cadre de la présente invention. A titre d'exemple de ligands, on peut citer le tris[2-diphénylephosphino)éthyle]phosphine ($PP_3$), le carbène A, la tricyclohexylphosphine.

Les catalyseurs peuvent, le cas échéant, être immobilisés sur des supports hétérogènes afin d'assurer une séparation facile dudit catalyseur et/ou son recyclage. Lesdits supports hétérogènes peuvent être choisis parmi les supports à base de gel de silice ou de polymères plastiques comme, par exemple, le polystyrène ; les supports carbonés choisis parmi les nanotubes de carbone ; le carbure de silice ; l'alumine ; ou le chlorure de magnésium ($MgCl_2$).

Dans le procédé selon l'invention, la réaction peut se produire sous une pression de $CO_2$, en faisant barboter du $CO_2$ dans le milieu réactionnel ou sous une atmosphère sèche contenant du $CO_2$ (air ambiant séché comprenant, par exemple environ 78% en volume d'azote, 21% en volume d'oxygène, et d'environ de 0,2 à 0,04% en volume de dioxyde de carbone). La réaction peut également se produire en utilisant du $CO_2$ supercritique.

De préférence, la réaction se produit sous une pression de $CO_2$.

La pression du $CO_2$, peut alors être comprise entre 1 et 50 bars, de préférence entre 1 et 30, plus préférentiellement entre 1 et 10 bars, bornes incluses.

La température de la réaction peut être comprise entre 25 et 150°C, de préférence entre 50 et 125°C, plus préférentiellement entre 70 et 100°C, bornes incluses.

La durée de la réaction dépend du taux de conversion de l'amine de formule (II). La réaction est avantageusement maintenue jusqu'à la conversion totale de l'amine de formule (II). La réaction est effectuée pendant une durée de 5 minutes à 72 heures, avantageusement de 15 minutes à 48 heures, de préférence de 1 à 48 heures, bornes incluses.

Le procédé de l'invention, en particulier la réaction entre les différents réactifs, peut avoir lieu dans un ou un mélange d'au moins deux solvant(s) choisi(s) parmi :

- les éthers, de préférence, l'éther diéthylique, ou le THF ;
- les hydrocarbures, de préférence, le benzène, ou le toluène ;
- les solvants azotés, de préférence, la pyridine, ou l'acétonitrile ;
- les sulfoxydes, de préférence, le diméthylesulfoxyde ;
- les halogénures d'alkyle, de préférence, le chloroforme, ou le chlorure de méthylène.

Selon une variante préférée de l'invention, il n'est pas nécessaire d'ajouter un solvant supplémentaire. Dans ce cas l'amine de formule (II) est le solvant. Ainsi, outre son rôle de fonctionnalisation du $CO_2$, l'amine sert de solvant.

Le rapport molaire entre le composé silane de formule (III) et l'amine de formule (II) est de 1 à 10, de préférence de 1 à 3, bornes incluses.

La quantité de catalyseur est de 0,001 à 1 équivalent molaire, de préférence de 0,01 à 1 équivalent molaire, plus préférentiellement de 0,01 à 0,9 équivalent molaire, encore plus préférentiellement de 0,01 à 0,5 équivalent molaire, bornes incluses, par rapport à l'amine de formule (II).

Les différents réactifs utilisés dans le procédé de l'invention (les amines de formule (II), les agents nucléophiles de formule (IV), les composés silane de formule (III), les (pré-)catalyseurs etc.) sont, en général, des composés commerciaux ou peuvent être préparés par tout procédé connu de l'homme du métier.

Comme déjà indiqué, l'invention concerne également, le procédé de préparation de composés azotés marqués de formule (I') :

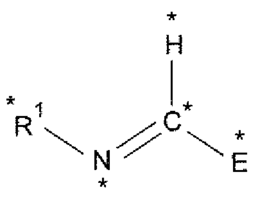

(I')

dans laquelle

- $R^{1*}$ est tel que défini précédemment pour $R^1$, et comporte éventuellement un $H^*$, $C^*$, $N^*$, $O^*$, $F^*$, $Si^*$ et/ou $S^*$ tels que définis ci-dessous ;
- $E^*$ représente $R^{5*}R^{6*}N^*$-, $R^{7*}O^*$-, avec

- $R^{5*}$, $R^{6*}$ et $R^{7*}$ tels que définis précédemment pour $R^5$, $R^6$ et $R^7$,
- O* représentant un atome d'oxygène ($^{16}O$), un isotope $^{17}O$ ou $^{18}O$, et
- N* représentant un atome d'azote ($^{14}N$), un isotope $^{15}N$ ;

- H* représente un atome d'hydrogène ($^1H$), le deutérium ($^2H$) ou le tritium ($^3H$) ;
- C* représente un atome de carbone ($^{12}C$), un isotope $^{11}C$, $^{13}C$ ou $^{14}C$;
- N* représente un atome d'azote ($^{14}N$), un isotope $^{15}N$ ;
- O* représente un atome d'azote ($^{16}O$), un isotope $^{18}O$ ;
- F* représente un atome d'azote ($^{19}F$), un isotope $^{18}F$ ;
- Si* représente un atome de silicium ($^{28}Si$), un isotope $^{29}Si$ ou $^{30}Si$ ;
- S* représente un atome de soufre ($^{32}S$), un isotope $^{33}S$, $^{34}S$ ou $^{36}S$ ;

caractérisé en ce que l'on fait réagir une amine de formule (II') :

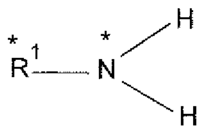

(II')

dans laquelle $R^{1*}$ et N* sont est tel que défini ci-dessus ; avec du $C^*O_2^*$ dans lequel C* et O* sont tels que définis ci-dessus ; en présence
d'un catalyseur,
d'un composé silane de formule (III')

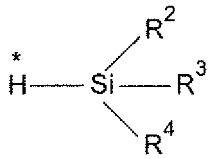

(III')

dans laquelle

- $R^2$, $R^3$, $R^4$ et H* sont tels que définis ci-dessus, et

d'un agent nucléophile de formule (IV) :

E*-H (IV')

dans laquelle E* est tel que défini ci-dessus et H est un atome d'hydrogène.

Les composés de formule (I') correspondent en fait aux composés de formule (I) comportant au moins un radiomarqueur/radiotraceur ou un isotope choisi.

Par isotopes on entend, pour un même élément, deux atomes ayant le même nombre de protons (et d'électrons) mais un nombre de neutrons différent. Ayant le même nombre d'électrons et de protons, les propriétés chimiques des isotopes d'un même élément sont presque identiques. Il peut exister, cependant, de légères variations de la vitesse d'une réaction chimique lorsqu'un des atomes d'un réactif est remplacé par un de ses isotopes. En revanche, comme le noyau ne comporte pas le même nombre de neutrons, la masse des atomes varie ce qui peut rendre l'atome instable : c'est pourquoi ils peuvent être radioactifs. Il s'agit alors de radioisotopes. Dans le cadre de l'invention, le terme « isotopes » peut également englober les « radioisotopes ».

Le radiomarquage est le fait d'associer à une molécule ou un composé donnés un isotope qui permettra de suivre l'évolution ou/et la fixation des molécules, par exemple, dans un organe. Le radiotraceur est le ou le(s) élément(s) radioactif(s) présent(s) au sein d'une molécule pour suivre le cheminement de cette substance, par exemple, dans un organe.

Le procédé de l'invention peut être réalisé avec des radioisotopes et/ou des isotopes stables portés par les différents réactifs comme indiqué ci-dessus. Il peut ainsi permettre l'accès aux composés azotés marqués $^{11}C$, $^{13}C$, $^{14}C$, $^{15}N$, $^2H$ (D), $^3H$ (T), $^{17}O$ et/ou $^{18}O$.

Une application directe de procédé peut alors être la synthèse de formamidines, ou des hétérocycles azotés

tels que les benzimidazoles, benzoxazoles, quinazolinones, 3,4-dihydroquinazolines, etc. marqués incorporant des radioisotopes et/ou isotopes stables en utilisant des réactifs marqués. Cette méthode permettrait ainsi l'accès aux formamidines marquées $^{11}C$, $^{13}C$, $^{14}C$, $^{15}N$, $^{2}H$ (D) et $^{3}H$ (T), $^{17}O$ et $^{18}O$ comme montré en Figure 1.

L'utilisation de molécules à des fins de traçage, de métabolisation, d'imagerie, etc., est détaillée dans la littérature (U. Pleiss, R. Voges, "Synthesis and Applications of Isotopically Labelled Compounds, Volume 7". Wiley-VCH, 2001 ; R. Voges, J. R. Heys, T. Moenius, "Préparation of Compounds Labeled with Tritium and Carbon-14". Wiley-VCH: Chippenham (UK), 2009). La possibilité de former les composés formamides marqués peut être assurée par la disponibilité des réactifs marqués correspondant, par exemple, par :

- les amines $R^1NH_2$ enrichies en $^{15}N$ sont accessibles à partir du chlorure d'ammonium enrichi $^{15}N$ ; $[^{15}NH_4][Cl]$ (Yong-Joo Kim, Max P. Bernstein, Angela S. Galiano Roth, Floyd E. Romesberg, Paul G. Williard, David J. Fuller, Aidan T. Harrison, and David B. Collum, J. Org. Chem. 1991, 56, p. 4435-4439) ;
- les amines $R^1NH_2$ avec $R^1$ marqués sont préparées par les voies de synthèses détaillées par U. Pleiss, R. Voges, "Synthesis and Applications of Isotopically Labelled Compounds, Volume 7", Wiley-VCH, 2001 ; et R. Voges, J. R. Heys, T. Moenius, "Préparation of Compounds Labeled with Tritium and Carbon-14", Wiley-VCH: Chippenham (UK), 2009) ;
- le $CO_2$ marqué $^{11}C$ ou $^{14}C$ est la source principale de $^{11}C$ et $^{14}C$ est obtenue par acidification du carbonate de baryum marqué $Ba^{14}CO_3$ (R. Voges, J. R. Heys, T. Moenius, "Preparation of Compounds Labeled with Tritium and Carbon-14". Wiley-VCH: Chippenham (UK), 2009) ;
- le $CO_2$ marqué $^{17}O$ ou $^{18}O$ est utilisé en chimie de synthèse pour produire des molécules marquées $^{17}O$ et $^{18}O$ (Christopher J. Dinsmore and Swati P. Mercer, Organic Letters, 2004 Vol., 6, No. 17 2885-2888 ; John T. Groves and Yoshihito Watanabe, J. Am. Chem. Soc., 1988,110, p. 8443-8452) ;
- les alcools marqués $^{17}O$ ou $^{18}O$ est utilisé en chimie de synthèse pour produire des molécules marquées $^{17}O$ et $^{18}O$ (Christopher J. Dinsmore and Swati P. Mercer, Organic Letters, 2004 Vol. 6, No. 17 2885-2888. John T. Groves and Yoshihito Watanabet, J. Am. Chem. SOC. 1988, 110, 8443-8452) ;
- les silanes $R^3R^4R^5Si$-H marqués $^{2}H$ (deutérium ou D) ou $^{3}H$ (tritium ou T) sont accessibles à partir du chlorosilane correspondant $R^3R^4R^5Si$-Cl et d'hydrure de lithium (LiH) ou tétrahydruroaluminate de lithium ($LiAlH_4$), les hydrures étant disponibles tous deux en versions deutérée et tritiée (T. A. Kochina, D. V. Vrazhnov, E. N. Sinotova, V. V. Avrorin, M. Yu. Katsap, and Yu. V. Mykhov, Russian Journal of General Chemistry, Vol. 72, No. 8, 2002, p. 1222-1224 ; E. A. Shishigin, V. V. Avrorin, T. A. Kochina, and E. N. Sinotova, Russian Journal of General Chemistry, Vol. 75, No. 1, 2005, p. 152).

Si le marquage de composés azotés est possible avec les noyaux $^{11}C$, $^{13}C$, $^{14}C$, $^{15}N$, $^{2}H$ (D), $^{3}H$ (T), $^{17}O$ et $^{18}O$, l'application visant à la formation de molécules marquées $^{14}C$ peut être la plus prometteuse en termes d'impact et de demande.

Les molécules marquées $^{14}C$ ont contribué à de nombreuses avancées dans les sciences du vivant (mécanismes enzymatiques, mécanismes biosynthétiques, biochimie), les sciences de l'environnement (traçage de déchets), la recherche (élucidation de mécanismes réactionnels) ou encore le diagnostic, la recherche et le développement de nouveaux produits pharmaceutiques et thérapeutiques. Les molécules marquées $^{14}C$ présentent, en effet, un avantage pour les études métaboliques car le $^{14}C$ est facilement détectable et quantifiable en milieu *in vitro* comme *in vivo.*

La source principale de $^{14}C$ est le $^{14}CO_2$ qui est obtenu par acidification du carbonate de baryum $Ba^{14}CO_3$. La mise au point de procédés de synthèse de molécules de base servant à l'élaboration de médicaments est donc primordiale pour produire des principes actifs marqués $^{14}C$ dont le métabolisme pourra ainsi être déterminé (R. Voges, J. R. Heys, T. Moenius, "Preparation of Compounds Labeled with Tritium and Carbon-14". Wiley-VCH: Chippenham (UK), 2009).

La contrainte majeure limitant la synthèse de molécules marquées $^{14}C$ est la nécessité d'avoir un rendement élevé en produit $^{14}C$ formé par rapport à la quantité de $^{14}CO_2$ utilisée et de reposer sur un nombre restreint d'étapes afin de limiter au maximum les coûts liés à l'utilisation de $Ba^{14}CO_3$ (U. Pleiss, R. Voges, "Synthesis and Applications of Isotopically Labelled Compounds, Volume 7". Wiley-VCH, 2001 ; R. Voges, J. R. Heys, T. Moenius, "Preparation of Compounds Labeled with Tritium and Carbon-14". Wiley-VCH: Chippenham (UK), 2009).

Le procédé selon l'invention répond à ces exigences car la pression de travail en $CO_2$ peut être faible, par exemple de 0,2 à 1 bar. En outre, le taux d'incorporation en $CO_2$ (ou rendement par rapport au $CO_2$ introduit) reste élevé, et peut, par exemple, dépasser les 95%.

Les conditions de température, de durée de réaction, de solvant, ainsi que les quantités de réactifs et de catalyseurs mises en oeuvre dans le procédé de préparation de composés azotés marqués de formule (I') sont celles décrites précédemment dans le cadre du procédé de préparation de composés azotés de formule (I).

Enfin, la synthèse de composés azotés marqués $^{14}C$ selon le procédé de l'invention, est une amélioration très nette par rapport aux technologies connues car il permet d'accéder aux composés azotés en une seule étape à partir

du $CO_2$ avec de bons rendements et une bonne sélectivité. L'intérêt des composés azotés marqués $^{14}C$ pour la synthèse de molécules complexes marquées $^{14}C$ est illustrée dans les références suivantes dans le cas de principes actifs pharmaceutiques : J. Z. Ho and coll, Helvetica Chimica Acta, 2005, 88, p.1040 ; I. V. Ekhato, S. Bonacorsi Jr., J., Label Compd. Radiopharm, 2011, 54, p.202-205 ; Kenneth K. Chan, James A. Staroscik, Journal of Medicinal Chemistry, 1977, Vol. 20, No. 4, p.598.

Dans cette variante du procédé selon l'invention, lorsque la réaction se produit sous une pression de $CO_2$, la pression du $CO_2$, peut alors être comprise entre 0,2 et 50 bars, de préférence entre 0,2 et 30, plus préférentiellement entre 0,2 et 10 bars, bornes incluses.

L'invention a également pour objet l'utilisation du procédé de préparation de composés azotés de formule (I) selon l'invention, dans la fabrication de vitamines, de produits pharmaceutiques, de colles, de fibres acryliques et cuirs synthétiques, de pesticides, d'herbicides, d'antifongiques, et d'engrais.

L'invention a, en outre, pour objet un procédé de fabrication de vitamines, de produits pharmaceutiques, de colles, de fibres acryliques, de cuirs synthétiques, de pesticides, d'herbicides, d'antifongiques, et d'engrais, caractérisé en ce qu'il comprend une étape de préparation de composés azotés de formule (I) par le procédé selon l'invention.

Un autre objet de l'invention consiste en un procédé de fabrication de traceurs et de radiotraceurs, caractérisé en ce qu'il comprend une étape de préparation de composés azotés marqués de formule (I) et (I') par le procédé selon l'invention.

Comme déjà indiqué, le procédé selon l'invention conduit à la formation de composés azotés, en une seule étape, avec un bon rendement (allant jusqu'à 100%). Une simple filtration peut permettre de récupérer le catalyseur éventuellement supporté et d'éliminer les éventuels sous-produits silylés formés.

D'autres avantages et caractéristiques de la présente invention apparaîtront à la lecture des exemples ci-dessous donnés à titre illustratif et non limitatif et des Figures annexées dans lesquelles :

- la Figure 1 représente schématiquement la synthèse de formamidines marqués par des radioisotopes et/ou isotopes stables ($^{11}C$, $^{13}C$, $^{14}C$, $^{15}N$, $^{2}H$ (D), $^{3}H$ (T), $^{17}O$ et/ou $^{18}O$ avec le procédé de l'invention.

## EXEMPLES

### EXEMPLE 1 :

Le procédé de préparation de composés azotés de formule (I), peut être effectué selon le protocole expérimental suivant.

Les réactifs utilisés, notamment l'amine $R^1NH_2$, le (pré-)catalyseur, l'agent nucléophile EH et le composé silane, sont des produits commercialisés par Sigma-Aldrich, Acros et Alfa Aesar.

Sous atmosphère inerte, en boîte à gants, l'amine $R^1RNH_2$ (1 équivalent molaire), le (pré-)catalyseur (de 0,001 à 1 équivalent molaire), le composé silane (1 équivalent) et le solvant sont introduits dans un tube de Schlenk qui est ensuite scellé par un robinet J. Young. La concentration en amine et en composé silane dans le mélange réactionnel est d'environ 1M (concentration calculée sur la base du volume de solvant introduit). L'ordre d'introduction des réactifs n'a pas d'importance.

Le tube de Schlenk est ensuite mis sous pression de $CO_2$ (de 1 à 3 bar) à l'aide d'une rampe à vide puis est chauffé à une température comprise entre 25 et 100 °C jusqu'à la conversion totale de l'amine (de 5 minutes à 72 heures de réaction).

La réaction une fois terminée, les composés volatils sont éliminés sous pression réduite et le mélange réactionnel est purifié par chromatographie sur gel de silice. L'utilisation de THF comme éluant permet de récupérer les éventuels sous-produits silylés (mélange de siloxanes et silanols). Dans un deuxième temps, l'acétate d'éthyle est utilisé comme éluant pour récupérer le composé azoté. L'acétate d'éthyle contenu dans la solution ainsi collectée est alors éliminé sous pression réduite de façon à obtenir le composé azoté analytiquement pur.

Un ensemble de résultats est présenté ci-dessous, donnant des exemples de conversions d'amines en composés azotés (déterminées par RMN et/ou GC/MS). Les classes de produits obtenues dépendent de la nature de l'agent nucléophile utilisé. Les différentes classes sont répertoriées dans différents tableaux ci-dessous. Les structures des amines, de l'agent nucléophile, des silanes et des (pré-)catalyseurs testés sont représentées à chaque fois.

Le schéma réactionnel général est le suivant :

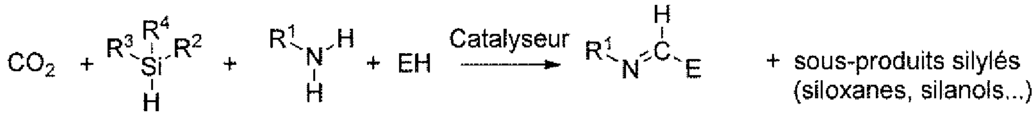


EH = $R^5R^6NH$ ou $R^7OH$ ou $R^8SH$

### A- Synthèse de benzimidazoles et dérivés à partir de diamines 1,2-aromatiques

Les premiers résultats présentés décrivent la synthèse de cycles benzimidazoles et dérivés, à partir de diamines 1,2-aromatiques. Dans ce cas, l'amine $R^1NH_2$ et l'agent nucléophile azoté $R^5R^6NH$ sont deux fonctions réactives d'une même molécule (diamine) et sont donc reliées par un lien covalent. Ce lien est préférentiellement un cycle aromatique de type benzène, pyridine ou pyrimidine et le cycle formé au cours de la réaction est un hétérocycle azoté à 5 atomes de type imidazole. Dans le cas où le nucléophile est oxygéné ($R^7OH$), les cycles obtenus sont des benzoxazoles. Les résultats présentés ont été réalisés en utilisant préférentiellement deux sources de réducteurs différents, le polyméthylhydrosiloxane (PMHS), commercialisé par Aldrich sous la référence 176206, et le phénylsilane ($PhSiH_3$) commercialisé par Aldrich. Dans le cas du PMHS, le silane étant un polymère, le nombre d'équivalents introduit est donné par rapport au nombre d'hydrures introduits et donc du nombre de monomères introduits par rapport à l'amine. Ainsi, l'introduction de 3 équivalents de PMHS correspond à l'introduction de 3 équivalents d'hydrure et donc 3 équivalents de monomères du PMHS par rapport à l'amine. Différents (pré)catalyseurs ont été testés pour la réaction. Le schéma réactionnel général est le suivant :

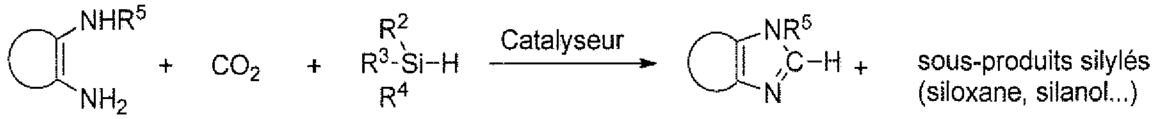


Les résultats sont indiqués dans le tableau 1. Dans tous les exemples du tableau 1, le solvant utilisé est le THF.

Tableau 1 :

| Amine | Silane (éq) | (Pré-) Catalyseur | Equivalence Catalyseur | Température | Temps | Conversion |
|---|---|---|---|---|---|---|
| 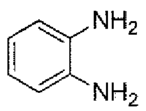 | PMHS 3éq | Carbène A | 5%mol | 70 °C | 24 h | 90% |
| | $PhSiH_3$ 1éq | Carbène A | 5%mol | 70 °C | 24 h | 85% |
| | PMHS 3 éq | $Cu(OAc)_2(H_2O)$ + dppb | 5%mol | 100 °C | 24 h | 35% |
| 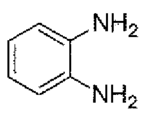 | PMHS 3éq | Carbène A | 5%mol | 70 °C | 24 h | 90% |
| | $PhSiH_3$ 1éq | Carbène A | 5%mol | 70 °C | 24 h | 85% |
| 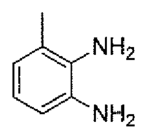 | PMHS 3éq | Carbène A | 5%mol | 70 °C | 24 h | 79% |
| 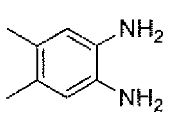 | PMHS 3éq | Carbène A | 5%mol | 70 °C | 24 h | 50% |
| | PhSiH3 1éq | Carbène A | 5%mol | 70 °C | 24 h | 35% |

(suite)

| Amine | Silane (éq) | (Pré-) Catalyseur | Equivalence Catalyseur | Température | Temps | Conversion |
|---|---|---|---|---|---|---|
| 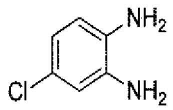 | PMHS 3éq | Carbène A | 5%mol | 70 °C | 24 h | 55% |
| 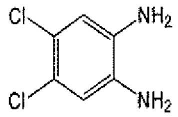 | PhSiH3 1éq | Carbène A | 5%mol | 70 °C | 24 h | 40% |
| 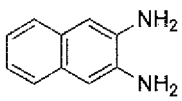 | PMHS 3éq | Carbène A | 5%mol | 70 °C | 24 h | 39% |
| | $PhSiH_3$ 1éq | Carbène A | 5%mol | 70 °C | 24 h | 67% |
| 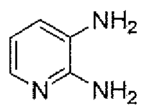 | PMHS 3éq | Carbène A | 5%mol | 70 °C | 24 h | 35% |
| | $PhSiH_3$ 1éq | Carbène A | 5%mol | 70 °C | 24 h | 72% |
| 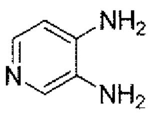 | PMHS 3éq | Carbène A | 5%mol | 70 °C | 24 h | 21% |
| | PMHS 3éq | Carbène A | 5%mol | 100 °C | 24 h | 40% |
| | $PhSiH_3$ 1éq | Carbène A | 5%mol | 70 °C | 24 h | 50% |
| 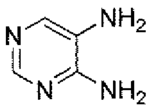 | $PhSiH_3$ 1éq | Carbène A | 5%mol | 70 °C | 24 h | 28% |
| 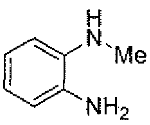 | PMHS 3éq | Carbène A | 5%mol | 70 °C | 24 h | 74% |
| 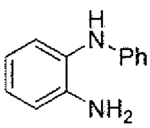 | PMHS 3éq | Carbène A | 5%mol | 70 °C | 24 h | 72% |
| | $PhSiH_3$ 1éq | Carbène A | 5%mol | 70 °C | 24 h | 65% |
| 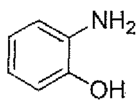 | PMHS 3éq | Carbène A | 5%mol | 70 °C | 24 h | 14% |

Les résultats montrent que, dans les conditions opératoires indiquées dans le tableau 1, le $CO_2$ peut être transformé avec de bons voire très bons (>70%) rendements en hétérocycles azotés dans lesquelles toutes les liaisons C=O du $CO_2$ ont été remplacées, en présence d'un catalyseur organique.

**B- Synthèse de dérivés 4-quinazolinones à partir d'amines aromatiques fonctionnalisées**

Les résultats suivants décrivent la synthèse de dérivés 4-quinazolinones à partir d'amines aromatiques fonctionnalisées en position ortho par des amides (anthranilamides). Dans ce cas, l'amine $R^1NH_2$ et le nucléophile azoté $R^5R^6NH$ (ici un amide) sont deux fonctions réactives d'une même molécule (diamine) et sont donc reliées par un lien covalent. Ce lien est préférentiellement un cycle aromatique de type benzène, pyridine ou pyrimidine et le cycle formé

au cours de la réaction est un hétérocycle azoté à 6 atomes. Les résultats présentés dans le tableau 2 ont été obtenus en utilisant préférentiellement le polyméthylhydrosiloxane (PMHS) comme réducteur, celui-ci se montrant plus efficace sur ces réactifs que le phénylsilane ($PhSiH_3$).

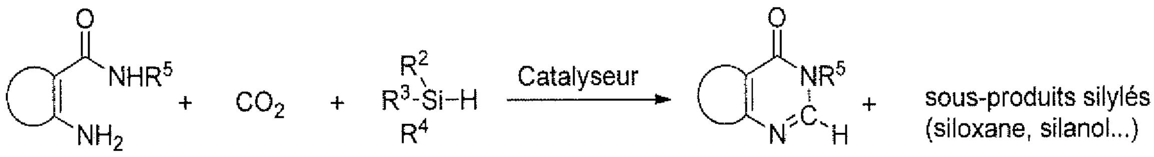


Les résultats, dans les conditions opératoires décrites, sont indiqués dans le tableau 2. Dans tous les exemples du tableau 2, le solvant utilisé est le THF.

**Tableau 2 :**

| Amine | Silane (éq) | Catalyseur | Equivalence Catalyseur | Température | Temps | Conversion |
|---|---|---|---|---|---|---|
| 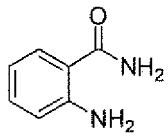 | PMHS 3éq | Carbène A | 5%mol | 70 °C | 24 h | 65% |
| | PMHS 6éq | Carbène A | 5%mol | 70 °C | 24 h | 85% |
| | $PhSiH_3$ 1éq | Carbène A | 5%mol | 100 °C | 24 h | 50% |
| | PMHS 3éq | $Cu(OAc)_2(H_2O)$ + dppb | 5%mol | 100°C | 24 h | 30% |
| 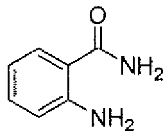 | PMHS 3éq | Carbène A | 5%mol | 70 °C | 24 h | 65% |
| | PMHS 6éq | Carbène A | 5%mol | 70 °C | 24 h | 85% |
| | $PhSiH_3$ 1éq | Carbène A | 5%mol | 100 °C | 24 h | 50% |
| 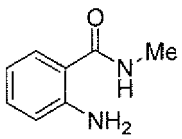 | PMHS 3éq | Carbène A | 5%mol | 70 °C | 24 h | 27% |
| 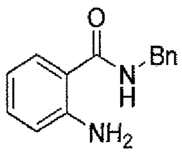 | PMHS 3éq | Carbène A | 5%mol | 70 °C | 24 h | 34% |
| 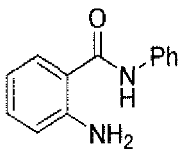 | PMHS 3éq | Carbène A | 5%mol | 70 °C | 24 h | 56% |

Ces dérivés 4-quinazolinones présentent un intérêt synthétique particulier puisqu'ils constituent des motifs couramment employés pour le développement de médicaments.

**C- synthèse de dérivés 3,4-dihydroquinazolines à partir de 2-aminobenzylamines**

Les résultats suivants décrivent quant à eux la synthèse de dérivés 3,4-dihydroquinazolines à partir de 2-aminobenzylamines. Dans ce cas, l'amine $R^1NH_2$ et le nucléophile azoté $R^5R^6NH$ sont deux fonctions réactives d'une même molécule (diamine) et sont donc reliées par un lien covalent et le cycle formé au cours de la réaction est un

hétérocycle azoté à 6 atomes. Les résultats présentés dans le tableau 3 ont été obtenus en utilisant préférentiellement deux sources de réducteurs différents, le polyméthylhydrosiloxane (PMHS) et le phénylsilane ($PhSiH_3$).

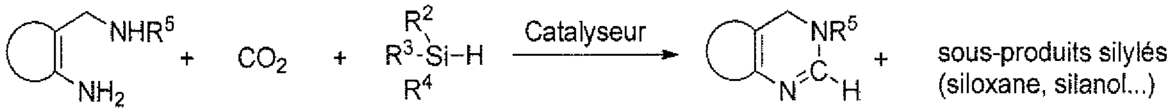


Les résultats sont indiqués dans le tableau 3. Dans tous les exemples du tableau 3, le solvant utilisé est le THF.

**Tableau 3 :**

| Amine | Silane (éq) | Catalyseur | Equivalence Catalyseur | Température | Temps | Conversion |
|---|---|---|---|---|---|---|
| 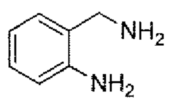 | PMHS 3éq | Carbène A | 5%mol | 70 °C | 24 h | 33% |
| | $PhSiH_3$ 1éq | Carbène A | 5%mol | 100 °C | 24 h | 5% |
| | PMHS 3éq | $Cu(OAc)_2$ ($H_2O$) + dppb | 5%mol | 100 °C | 24 h | 10% |
| 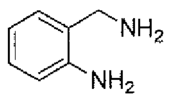 | PMHS 3éq | Carbène A | 5%mol | 70 °C | 24 h | 33% |
| | $PhSiH_3$ 1éq | Carbène A | 5%mol | 100 °C | 24 h | 5% |
| 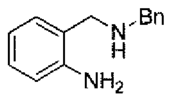 | PMHS 3éq | Carbène A | 5%mol | 70 °C | 24 h | 60% |
| 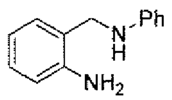 | PMHS 3éq | Carbène A | 5%mol | 70 °C | 24 h | 56% |

Les résultats montrent que, dans les conditions opératoires indiquées dans le tableau 3, il est possible de former, selon le procédé de l'invention, des hétérocycles azotés non-aromatiques.

**D- Synthèse de la 8-aminonaphtylamine**

Le procédé de l'invention a été utilisé pour la synthèse de la 8-aminonaphtylamine selon les conditions opératoires indiqués dans le schéma suivant. La particularité de l'amine de départ est que les fonctions amines sont portées par deux noyaux aromatiques différents. Le silane utilisé est le phénylsilane ($PhSiH_3$).

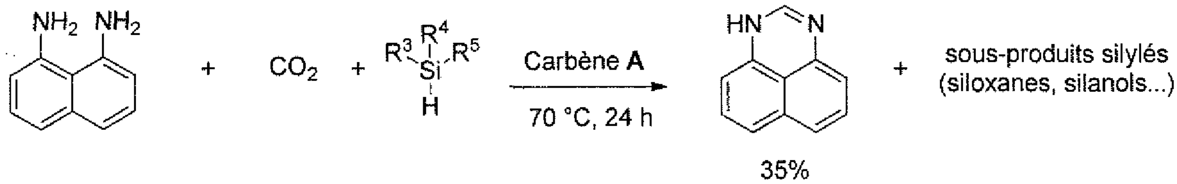


**E- Synthèse de dérivés formamidines à partir de mélanges d'amines**

Les résultats suivants décrivent quant à eux la synthèse de dérivés formamidines à partir de mélanges d'amines. Dans ce cas, l'amine $R^1NH_2$ et l'agent nucléophile $R^5R^6NH$ sont deux molécules distinctes, qui peuvent être identiques ou non pour obtenir des formamidines symétriques ou non. Les résultats présentés dans le tableau 4 ont été obtenus en utilisant préférentiellement le phénylsilane ($PhSiH_3$).

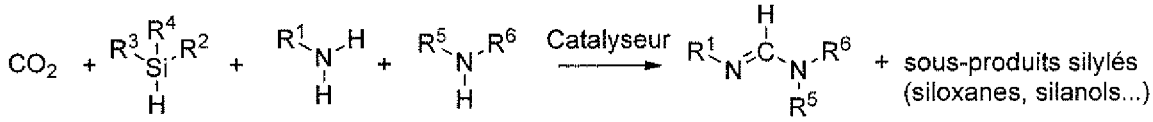


Les résultats sont indiqués dans le tableau 4. Le solvant utilisé dans cet exemple est le THF.

**Tableau 4 :**

| Amine | Silane (éq) | Catalyseur | Equivalence Catalyseur | Température | Temps | Conversion en formamidine PhN=CH-NHPh |
|---|---|---|---|---|---|---|
| 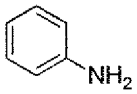 | $PhSiH_3$ 1éq | TBD | 5%mol | 100°C | 24 h | 28% |
| | $PhSiH_3$ 1éq | TBD | 5%mol | 100°C | 48 h | 30% |
| | $PhSiH_3$ 0,6éq | TBD | 5%mol | 100°C | 48 h | 44% |
| | $PhSiH_3$ 1éq | $Cu(OAc)_2$. $(H_2O)$+dppb | 5%mol | 100°C | 24h | 36% |
| | PMHS 3éq | $Cu(OAc)_2$ $(H_2O)$+dppb | 5%mol | 100°C | 24h | 70% |
| | $PhSiH_3$ 1éq | $Fe(BF_4)_2$ $(H_2O)_6$+$PP_3$ | 5%mol | 100°C | 24h | 26% |
| | $PhSiH_3$ 2éq | $Fe(acac)_3$+ $PP_3$ | 5%mol | 100°C | 24h | 15% |
| | $PhSiH_3$ 2éq | $ZnEt_2$ | 10%mol | 100°C | 24h | 6% |
| $C_7H_{15}NH_2$ | $PhSiH_3$ 1éq | $Fe(BF_4)_2$ $(H_2O)_6$+$PP_3$ | 5%mol | 100°C | 24h | 50% |
| 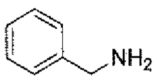 | $PhSiH_3$ 1éq | $Fe(BF_4)_2$ $(H_2O)_6$+$PP_3$ | 5%mol | 100°C | 24h | 52% |
| | $PhSiH_3$ 2éq | $Fe(acac)_3$+ $PP_3$ | 5%mol | 100°C | 24h | 5% |

Les résultats montrent que, dans les conditions opératoires indiquées dans le tableau 4, il n'est pas nécessaire que l'amine de formule (II) et l'agent nucléophile de formule (IV) soit portés par la même molécule. Il est ainsi possible de former, selon le procédé de l'invention, des formamidines non-cycliques.

Les abréviations utilisées dans ces exemples sont représentées ci-dessous :

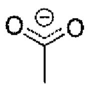
ligand OAc

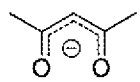
ligand acac

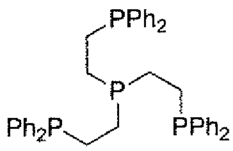
$PP_3$

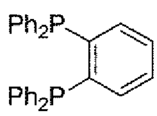
dppb

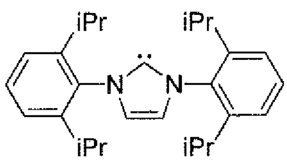
Carbène A

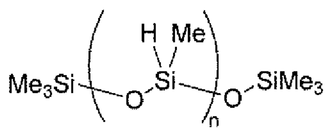
PMHS

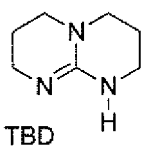
TBD

## Revendications

**1.** Procédé de préparation de composés azotés de formule (I) :

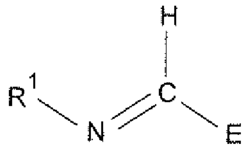

(I)

dans laquelle

- $R^1$ représente un atome d'hydrogène, un groupe alkyle, un groupe alcényle, un groupe alcynyle, un groupe aryle, un groupe hétéroaryle, un hétérocycle, un groupe carbonyle (-CO-), un groupe silylé, un groupe siloxy, un groupe amino, lesdits groupes alkyle, alcényle, alcynyle, aryle, hétéroaryle, hétérocycle, silylé, siloxy et amino étant éventuellement substitués,
- $R^1$ comporte éventuellement un H, C, N, O, F, Si et/ou S tels que définis ci-dessous ;
- H représente un atome d'hydrogène ($^1H$), le deutérium ($^2H$) ou le tritium ($^3H$) ;
- C représente un atome de carbone ($^{12}C$), un isotope $^{11}C$, $^{13}C$ ou $^{14}C$ ;
- N représente un atome d'azote ($^{14}N$), un isotope $^{15}N$ ;
- O représente un atome d'oxygène ($^{16}O$), un isotope $^{17}O$ ou $^{18}O$ ;
- F représente un atome de fluor ($^{19}F$), un isotope $^{18}F$ ;
- Si représente un atome de silicium ($^{28}Si$), un isotope $^{29}Si$ ou $^{30}Si$ ;
- S représente un atome de soufre ($^{32}S$), un isotope $^{33}S$, $^{34}S$ ou $^{36}S$ ;
- E représente un groupe $R^5R^6N$- ou $R^7O$- avec

  - $R^5$, $R^6$ et $R^7$ représentant, indépendamment l'un de l'autre, un atome d'hydrogène, un groupe alkyle, un groupe alcényle, un groupe alcynyle, un groupe aryle, un groupe hétéroaryle, un hétérocycle, un groupe (-(CO)-), un groupe silylé, un groupe siloxy, un groupe amino, lesdits groupes alkyle, alcényle, alcynyle, aryle, hétéroaryle, hétérocycle, silylé, siloxy et amino étant éventuellement substitués, ou
  - $R^5$ et $R^6$, pris ensemble avec l'atome d'azote auquel ils sont liés forment un hétérocycle éventuellement substitué, ou
  - $R^1$ et $R^5$, pris ensemble avec les deux atomes d'azote auxquels ils sont respectivement liés forment un hétérocycle éventuellement substitué, ou
  - $R^1$ et $R^7$, pris ensemble avec l'atome d'azote et l'atome d'oxygène auxquels ils sont respectivement liés forment un hétérocycle éventuellement substitué,
  - $R^5$, $R^6$ et $R^7$ comportent éventuellement un H, C, N, O, F, Si et/ou S tels que définis ci-dessus,
  - $R^1$, N et O étant tels que définis ci-dessus ;

**caractérisé en ce que** l'on fait réagir une amine de formule (II) :

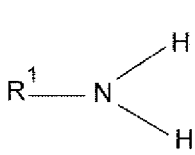

(II)

dans laquelle $R^1$ et N sont tels que définis ci-dessus ; avec du $CO_2$ dans lequel C et O sont tels que définis ci-dessus ; et en présence
d'un catalyseur,
d'un composé silane de formule (III) :

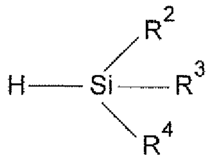

(III)

dans laquelle

- H est tel que défini ci-dessus,
- $R^2$, $R^3$ et $R^4$ représentent, indépendamment l'un de l'autre, un atome d'hydrogène, un atome d'halogène, un groupe hydroxyle, un groupe alkyle, un groupe alcényle, un groupe alcynyle, un groupe alkoxy, un groupe silylé, un groupe siloxy, un groupe aryle, un groupe amino, lesdits groupes alkyle, alcényle, alcynyle, alkoxy, silylé, siloxy, aryle et amino étant éventuellement substitués, ou
- $R^4$ est tel que défini ci-dessus et $R^2$ et $R^3$, pris ensemble avec l'atome de silicium auquel ils sont liés forment un hétérocycle silylé éventuellement substitué, et

d'un agent nucléophile de formule (IV) :

E-H (IV)

dans laquelle E est tel que défini ci-dessus et H est un atome d'hydrogène.

**2.** Procédé selon la revendication 1, **caractérisé en ce que** dans l'amine de formule (II), $R^1$ représente un atome d'hydrogène, un groupe alkyle, un groupe aryle, un groupe hétéroaryle, un hétérocycle, un groupe amino, lesdits groupes alkyle, amino, aryle, hétérocycle et hétéroaryle étant éventuellement substitués.

**3.** Procédé selon l'une des revendications 1 ou 2, **caractérisé en ce que** dans le composé silane de formule (III), $R^2$, $R^3$ et $R^4$ représentent, indépendamment l'un de l'autre, un atome d'hydrogène, un groupe alkyle, un groupe alkoxy, un groupe aryle, un groupe silylé, un groupe siloxy, lesdits groupes alkyle, alkoxy, silylé, siloxy et aryle étant éventuellement substitués.

**4.** Procédé selon l'une quelconque des revendications 1 à 3, **caractérisé en ce que** dans l'agent nucléophile E-H de formule (IV), E représente un groupe $R^5R^6N$- avec

- $R^5$ et $R^6$ représentant, indépendamment l'un de l'autre, un atome d'hydrogène, un groupe alkyle, un groupe aryle, un groupe hétéroaryle, un hétérocycle, un groupe amino, lesdits groupes alkyle, aryle, hétéroaryle, hétérocycle et amino étant éventuellement substitués, ou
- $R^5$ et $R^6$, pris ensemble avec l'atome d'azote auquel ils sont liés forment un hétérocycle éventuellement substitué.

**5.** Procédé selon l'une quelconque des revendications 1 à 4, **caractérisé en ce que** le catalyseur est choisi parmi les catalyseurs organiques ou les catalyseurs métalliques choisi parmi les sels ou les complexes métalliques.

**6.** Procédé selon la revendication 5, **caractérisé en ce que** le catalyseur organique est :

- une amine secondaire ou tertiaire choisie parmi le triazabicyclodécène (TBD) ; le N-méthyltriazabicyclodécène (MeTBD), le 1,8-diazabicyclo[5.4.0]undéc-7-ène (DBU), la triméthylamine, la triéthylemaine, la pipéridine, la 4-diméthylaminopyridine (DMAP), le 1,4-diazabicyclo[2.2.2]octane (DABCO), la proline, le phénylalanine, un sel de thiazolium, la *N*-diisopropyléthylamine (DIPEA ou DIEA) ; ou
- un carbène issu d'un sel d'imidazolium choisi parmi les sels de 1,3-bis(2,6-diisopropylphenyl)-1H-imidazol-3-ium (carbène A), 1,3-bis(2,6-diisopropylphenyl)-4,5-dihydro-1H-imidazol-3-ium (carbène C), 1,3-bis(2,4,6-triméthylphenyl)-1H-imidazol-3-ium (carbène B), 1,3-bis(2,4,6-triméthylphenyl)-4,5-dihydro-1H-imidazol-3-ium (carbène D), 4,5-dichloro-1,3-bis(2,6-diisopropylphenyl)-1H-imidazol-3-ium (carbène E), 1,3-di-tert-butyl-1H-imidazol-3-ium (carbène F), 1,3-di-tert-butyl-4,5-dihydro-1H-imidazol-3-ium, lesdits sels étant sous la forme de sels de chlorure.

**7.** Procédé selon la revendication 5, **caractérisé en ce que** le catalyseur métallique est choisi parmi les sels ou

complexes de :

- métaux choisis parmi le bore, le silicium, l'aluminium, le gallium, l'étain, l'indium ;
- métaux alcalins choisis parmi le sodium, le potassium ;
- métaux alcalinoterreux choisis parmi le magnésium, le calcium ;
- métaux de transition choisis parmi le nickel, le fer, le cobalt, le zinc, le cuivre, le rhodium, le ruthénium, le platine, le palladium, l'iridium ;
- terres rares choisis parmi le lanthane, le cérium, le praséodyme, le néodyme.

**8.** Procédé selon l'une quelconque des revendications 1 à 7, **caractérisé en ce que** la réaction est effectuée sous une pression de $CO_2$, comprise entre 1 et 50 bars, bornes incluses.

**9.** Procédé selon l'une quelconque des revendications 1 à 8, **caractérisé en ce que** la réaction est effectuée à une température comprise entre 25 et 150°C, bornes incluses.

**10.** Procédé selon l'une quelconque des revendications 1 à 9, **caractérisé en ce que** la réaction est effectuée pendant une durée de 5 minutes à 72 heures, bornes incluses.

**11.** Procédé selon l'une quelconque des revendications 1 à 10, **caractérisé en ce que** la réaction est effectuée dans un ou un mélange d'au moins deux solvant(s) choisi(s) parmi :

- les éthers choisis parmi l'éther diéthylique, ou le THF ;
- les hydrocarbures choisis parmi le benzène, ou le toluène ;
- les solvants azotés choisis parmi la pyridine, ou l'acétonitrile ;
- les sulfoxydes choisis parmi le diméthylesulfoxyde ;
- les halogénures d'alkyle choisis parmi le chloroforme, ou le chlorure de méthylène.

**12.** Procédé selon l'une quelconque des revendications 1 à 11, **caractérisé en ce que** le rapport molaire entre le composé silane de formule (III) et l'amine de formule (II) est de 1 à 10, de préférence de 1 à 3, bornes incluses.

**13.** Procédé selon l'une quelconque des revendications 1 à 12, **caractérisé en ce que** la quantité de catalyseur est de 0,001 à 1 équivalent molaire, bornes incluses, par rapport à l'amine de formule (II).

**14.** Utilisation d'un procédé de préparation de composés formamides de formule (I) selon l'une quelconque des revendications 1 à 13, dans la fabrication de vitamines, de produits pharmaceutiques, de colles, de fibres acryliques, de cuirs synthétiques, de pesticides, d'herbicides, d'antifongiques, et d'engrais.

**15.** Procédé de fabrication de traceurs et de radiotraceurs, **caractérisé en ce qu'**il comprend une étape de préparation de composés azotés de formule (I) par le procédé selon l'une quelconque des revendications 1 à 13.

## Patentansprüche

**1.** Verfahren zur Herstellung von Stickstoffverbindungen mit der folgenden Formel (I):

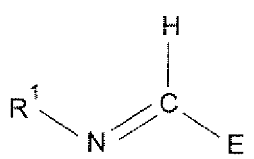

(I)

wobei

- $R^1$ ein Wasserstoffatom, eine Alkylgruppe, eine Alkenylgruppe, eine Alkynylgruppe, eine Arylgruppe, eine Heteroarylgruppe, einen Heterocyclus, eine Carbonylgruppe (-CO-), eine Silylgruppe, eine Siloxygruppe, eine Aminogruppe darstellt, wobei die Alkyl-, Alkenyl-, Alkynyl-, Aryl-, Heteroaryl-, Heterocyclus-, Silyl, Siloxy- und Aminogruppen eventuell substituiert sind,

- $R^1$ eventuell ein H, C, N, O, F, Si und/oder S umfasst, wie unten definiert;
- H ein Wasserstoffatom ($^1H$), Deuterium ($^2H$) oder Tritium ($^3H$) darstellt;
- C ein Kohlenstoffatom ($^{12}C$), ein Isotop $^{11}C$, $^{13}C$ oder $^{14}C$ darstellt;
- N ein Stickstoffatom ($^{14}N$), ein Isotop $^{15}N$ darstellt;
- 0 ein Sauerstoffatom ($^{16}O$), ein Isotop $^{17}O$ oder $^{18}O$ darstellt;
- Fein Fluoratom ($^{19}F$), ein Isotop $^{18}F$ darstellt;
- Si ein Siliciumatom ($^{28}Si$), ein Isotop $^{29}Si$ der $^{30}Si$ darstellt;
- S ein Schwefelatom ($^{32}S$), ein Isotop $^{33}S$, $^{34}S$ oder $^{36}S$ darstellt;
- E eine Gruppe $R^5R^6N$- oder $R^7O$- darstellt, wobei

  -- $R^5$, $R^6$ und $R^7$ unabhängig voneinander ein Wasserstoffatom, eine Alkylgruppe, eine Alkenylgruppe, eine Alkynylgruppe, eine Arylgruppe, eine Heteroarylgruppe, einen Heterocyclus, eine Gruppe (-(CO)-), eine Silylgruppe, eine Siloxygruppe, eine Aminogruppe darstellen, wobei die Alkyl-, Alkenyle, Alkynyl-, Aryl-, Heteroaryl-, Heterocyclus-, Silyl, Siloxy- und Aminogruppen eventuell substituiert sind, oder
  -- $R^5$ und $R^6$, zusammen mit dem Stickstoffatom, an das sie gebunden sind, einen Heterocyclus bilden, der eventuell substituiert ist, oder
  -- $R^1$ und $R^5$, zusammen mit den zwei Stickstoffatomen, an die sie jeweils gebunden sind, einen Heterocyclus bilden, der eventuell substituiert ist, oder
  -- $R^1$ und $R^7$, zusammen mit dem Stickstoffatom und dem Sauerstoffatom, an die sie jeweils gebunden sind, einen Heterocyclus bilden, der eventuell substituiert ist,
  -- $R^5$, $R^6$ und $R^7$ eventuell ein H, C, N, 0, F, Si und/oder S umfassen, wie oben definiert;
  -- $R^1$, N und 0 wie oben definiert sind;

**dadurch gekennzeichnet, dass** ein Amin mit der folgenden Formel (II) reagiert wird:

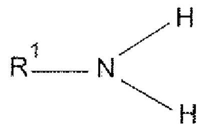

(II)

wobei $R^1$ und N wie oben definiert sind; mit $CO_2$, wobei C und 0 wie oben definiert sind; und in Anwesenheit eines Katalysators,
einer Silanverbindung mit der folgenden Formel (III) :

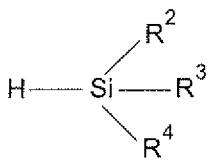

(III);

wobei

- H wie oben definiert ist,
- $R^2$, $R^3$ und $R^4$ unabhängig voneinander ein Wasserstoffatom, ein Halogenatom, eine Hydroxygruppe, eine Alkylgruppe, eine Alkenylgruppe, eine Alkynylgruppe, eine Alkoxygruppe, eine Silylgruppe, eine Siloxygruppe, eine Arylgruppe, eine Aminogruppe darstellt, wobei die Alkyl-, Alkenyl-, Alkynyl-, Alkoxy-, Silyl-, Siloxy-, Aryl- und Aminogruppen eventuell substituiert sind, oder
- $R^4$ wie oben definiert ist, und $R^2$ und $R^3$, zusammen mit dem Siliciumatom, an das sie gebunden sind, einen Silylheterocyclus bilden, der eventuell substituiert ist, und

ein nucleophiles Mittel mit der folgenden Formel (IV) :

E-H (IV)

wobei E wie oben definiert ist und H ein Wasserstoffatom ist.

2. Verfahren nach Anspruch 1, **dadurch gekennzeichnet, dass** im Amin mit der Formel (II) $R^1$ ein Wasserstoffatom, eine Alkylgruppe, eine Arylgruppe, eine Heteroarylgruppe, einen Heterocyclus, eine Aminogruppe darstellt, wobei die Alkyl-, Amino, Aryl-, Heterocyclus- und Heteroarylgruppen eventuell substituiert sind.

3. Verfahren nach einem der Ansprüche 1 oder 2, **dadurch gekennzeichnet, dass** in der Silanverbindung mit der Formel (III) $R^2$, $R^3$ und $R^4$ unabhängig voneinander ein Wasserstoffatom, eine Alkylgruppe, eine Alkoxygruppe, eine Arylgruppe, eine Silylgruppe, eine Siloxygruppe darstellen, wobei die Alkyl-, Alkoxy-, Silyl-, Siloxy- und Arylgruppen eventuell substituiert sind.

4. Verfahren nach einem der Ansprüche 1 bis 3, **dadurch gekennzeichnet, dass** im nucleophilen Mittel E-H mit der Formel (IV) E eine Gruppe $R^5R^6N$- darstellt, wobei

   - $R^5$ und $R^6$ unabhängig voneinander ein Wasserstoffatom, eine Alkylgruppe, eine Arylgruppe, eine Heteroarylgruppe, einen Heterocyclus, eine Aminogruppe darstellen, wobei die Alkyl-, Aryl-, Heteroaryl-, Heterocyclus- und Aminogruppen eventuell substituiert sind, oder
   - $R^5$ und $R^6$, zusammen mit dem Stickstoffatom, an das sie gebunden sind, einen Heterocyclus bilden, der eventuell substituiert ist.

5. Verfahren nach einem der Ansprüche 1 bis 4, **dadurch gekennzeichnet, dass** der Katalysator ausgewählt ist aus den organischen Katalysatoren oder den metallischen Katalysatoren, ausgewählt aus den Salzen oder den metallischen Komplexen.

6. Verfahren nach Anspruch 5, **dadurch gekennzeichnet, dass** organische Katalysator Folgendes ist:

   - ein sekundäres oder tertiärstes Amin, ausgewählt aus Triazabicyclodcen (TBD); N-Methyltriazabicyclodecen (MeTBD), 1,8-Diazabicyclo[5.4.0]undec-7-en (DBU), Trimethylamin, Triethylamin, Piperidin, 4-Dimethylaminopyridin (DMAP), 1,4-Diazabicyclo[2.2.2]octan (DABCO), Prolin, Phenylalanin, einem Thiazolsalz, *N*-Diisopropylethylamin (DIPEA oder DIEA); oder
   - ein Carben, das aus einem Imidazolsalz stammt, ausgewählt aus den Salzen von 1,3-bis(2,6-Diisopropylphenyl)-1H-imidazol-3-ium (Carben A), 1,3-bis(2,6-Diisopropylphenyl)-4,5-dihydro-1H-imidazol-3-ium (Carben C), 1,3-bis(2,4,6-Trimethylphenyl)-1H-imidazol-3-ium (Carben B), 1,3-bis(2,4,6-Trimethylphenyl)-4,5-dihydro-1H-imidazol-3-ium (Carben D), 4,5-Dichloro-1,3-bis(2,6-diisopropylphenyl)-1H-imidazol-3-ium (Carben E), 1,3-di-tert-Butyl-1H-imidazol-3-ium (Carben F), 1,3-di-tert-Butyl-4,5-dihydro-1H-imidazol-3-ium, wobei die Salze die Form von Chloridsalzen aufweisen.

7. Verfahren nach Anspruch 5, **dadurch gekennzeichnet, dass** der metallische Katalysator ausgewählt ist aus den Salzen oder den Komplexen von:

   - Metallen, ausgewählt aus Bor, Silicium, Aluminium, Gallium, Zinn und Indium;
   - Alkalimetallen, ausgewählt aus Natrium, Kalium;
   - Erdalkalimetallen, ausgewählt aus Magnesium, Calcium;
   - Übergangsmetallen, ausgewählt aus Nickel, Eisen, Kobalt, Zink, Kupfer, Rhodium, Ruthenium, Platin, Palladium, Iridium;
   - Seltenerden, ausgewählt aus Lanthan, Cer, Praseodym, Neodym.

8. Verfahren nach einem der Ansprüche 1 bis 7, **dadurch gekennzeichnet, dass** die Reaktion unter einem Druck von $CO_2$ durchgeführt wird, der zwischen 1 und 50 bar liegt, Grenzen eingeschlossen.

9. Verfahren nach einem der Ansprüche 1 bis 8, **dadurch gekennzeichnet, dass** die Reaktion bei einer Temperatur im Bereich zwischen 25 und 150 °C durchgeführt wird, Grenzen eingeschlossen.

10. Verfahren nach einem der Ansprüche 1 bis 9, **dadurch gekennzeichnet, dass** die Reaktion während einer Dauer von 5 Minuten bis 72 Stunden durchgeführt wird, Grenzen eingeschlossen.

11. Verfahren nach einem der Ansprüche 1 bis 10, **dadurch gekennzeichnet, dass** die Reaktion in einem oder einer Mischung von mindestens zwei Lösemittel(n) durchgeführt wird, ausgewählt aus:

   - Ethern, ausgewählt aus Diethylether oder THF;

- Kohlenwasserstoffen, ausgewählt aus Benzen oder Toluen;
- Stickstofflösemitteln, ausgewählt aus Pyridin oder Acetonitryl.
- Sulfoxiden, ausgewählt aus Dimethylsulfoxid;
- Alkylhalogeniden, ausgewählt aus Chloroform, oder Methylenchlorid.

**12.** Verfahren nach einem der Ansprüche 1 bis 11, **dadurch gekennzeichnet, dass** das Molverhältnis zwischen der Silanverbindung mit der Formel (III) und dem Amin mit der Formel (II) 1 bis 10, vorzugsweise 1 bis 3 ist, Grenzen eingeschlossen.

**13.** Verfahren nach einem der Ansprüche 1 bis 12, **dadurch gekennzeichnet, dass** die Menge des Katalysators von 0,001 bis 1, Grenzen eingeschlossen, mit Bezug auf das Amin mit der Formel (II) beträgt.

**14.** Verwendung eines Verfahrens zur Herstellung von Foramidverbindungen mit der Formel (I) nach einem der Ansprüche 1 bis 13 bei der Herstellung von Vitaminen, pharmazeutischen Produkten, Klebstoffen, Acrylfasern, synthetischem Ledern, Pestiziden, Herbiziden, Fungiziden und Düngemitteln.

**15.** Verfahren zur Herstellung von Tracern und radioaktiven Tracern, **dadurch gekennzeichnet, dass** es einen Schritt des Herstellens von Stickstoffverbindungen mit der Formel (I) durch das Verfahren nach einem der Ansprüche 1 bis 13 umfasst.

## Claims

**1.** Method for preparing nitrogenous compounds of formula (I) :

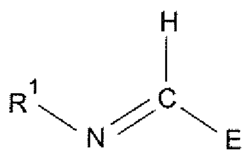

(I)

wherein

- $R^1$ represents a hydrogen atom, an alkyl group, an alkenyl group, an alkynyl group, an aryl group, a heteroaryl group, a heterocycle, a carbonyl group (-CO-), a silylated group, a siloxy group, an amino group, said alkyl, alkenyl, alkynyl, aryl, heteroaryl, heterocycle, silylated, siloxy and amino groups being optionally substituted,
- $R^1$ optionally comprises an H, C, N, O, F, Si and/or S as defined below;
- H represents a hydrogen atom ($^1H$), deuterium ($^2H$) or tritium ($^3H$) ;
- C represents a carbon atom ($^{12}C$), an isotope $^{11}C$, $^{13}C$ or $^{14}C$;
- N represents a nitrogen atom ($^{14}N$), an isotope $^{15}N$ ;
- 0 represents an oxygen atom ($^{16}O$), an isotope $^{17}O$ or $^{18}O$;
- F represents a fluorine atom ($^{19}F$), an isotope $^{18}F$;
- Si represents a silicon atom ($^{28}Si$), an isotope $^{29}Si$ or $^{30}Si$;
- S represents a sulphur atom ($^{32}S$), an isotope $^{33}S$, $^{34}S$ or $^{36}S$;
- E represents a group $R^5R^6N$- or $R^7O$- where
- $R^5$, $R^6$ and $R^7$ represent, independently from one another, a hydrogen atom, an alkyl group, an alkenyl group, an alkynyl group, an aryl group, a heteroaryl group, a heterocycle, a group (-(CO)-), a silylated group, a siloxy group, an amino group, said alkyl, alkenyl, alkynyl, aryl, heteroaryl, heterocycle, silylated, siloxy and amino groups being optionally substituted, or
- $R^5$ and $R^6$, taken together with the nitrogen atom to which they are bound form an optionally substituted heterocycle, or
- $R^1$ and $R^5$, taken together with the two nitrogen atoms to which they are respectively bound form an optionally substituted heterocycle, or
- $R^1$ and $R^7$, taken together with the nitrogen atom and the oxygen atom to which they are respectively bound form an optionally substituted heterocycle,
- $R^5$, $R^6$ and $R^7$ optionally comprise an H, C, N, O, F, Si and/or S as defined above,
- where $R^1$, N and O are as defined above;

**characterised in that** an amine of formula (II) is reacted:

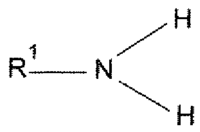

(II)

wherein $R^1$ and N are as defined above; with $CO_2$ wherein C and O are as defined above; and in the presence of a catalyst,
a silane compound of formula (III):

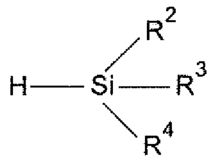

(III)

wherein

- H is as defined above,
- $R^2$, $R^3$ and $R^4$ represent, independently from one another, a hydrogen atom, a halogen atom, a hydroxyl group, an alkyl group, an alkenyl group, an alkynyl group, an alkoxy group, a silylated group, a siloxy group, an aryl group, an amino group, said alkyl, alkenyl, alkynyl, alkoxy, silylated, siloxy, aryl and amino groups being optionally substituted, or
- $R^4$ is as defined above and $R^2$ and $R^3$, taken together with the silicon atom to which they are bound form an optionally substituted silylated, heterocycle, and

a nucleophilic agent of formula (IV):

E-H (IV)

wherein E is as defined above and H is a hydrogen atom.

2. Method according to claim 1, **characterised in that** in the amine of formula (II), $R^1$ represents a hydrogen atom, an alkyl group, an aryl group, a heteroaryl group, a heterocycle, an amino group, said alkyl, amino, aryl, heterocycle and heteroaryl groups being optionally substituted.

3. Method according to one of claims 1 or 2, **characterised in that** in the silane compound of formula (III), $R^2$, $R^3$ and $R^4$ represent, independently of one another, a hydrogen atom, an alkyl group, an alkoxy group, an aryl group, a silylated group, a siloxy group, said alkyl, alkoxy, silylated, siloxy and aryl groups being optionally substituted.

4. Method according to any one of claims 1 to 3, **characterised in that** in the nucleophilic agent E-H of formula (IV), E represents a group $R^5R^6N$- where

   - $R^5$ and $R^6$ represent, independently of one another, a hydrogen atom, an alkyl group, an aryl group, a heteroaryl group, a heterocycle, an amino group, said alkyl, aryl, heteroaryl, heterocycle and amino groups being optionally substituted, or
   - $R^5$ and $R^6$, taken together with the nitrogen atom to which they are bound form an optionally substituted heterocycle.

5. Method according to any one of claims 1 to 4, **characterised in that** the catalyst is chosen from organic catalysts or metallic catalysts chosen from metallic salts or complexes.

6. Method according to claim 5, **characterised in that** the organic catalyst is:

   - a secondary or tertiary amine chosen from triazabicyclodecene (TBD); N-methyltriazabicyclodecene (MeTBD),

1,8-diazabicyclo[5.4.0]undec-7-ene (DBU), trimethylamine, triethylemaine, piperidine, 4-dimethylaminopyridine (DMAP), 1,4-diazabicyclo[2.2.2]octane (DABCO), proline, phenylalanine, a thiazolium salt, N-diisopropylethylamine (DIPEA or DIEA); or
- a carbene derived from an imidazolium salt chosen from the salts of 1,3-bis(2,6-diisopropylphenyl)-1H-imidazol-3-ium (carbene A), 1,3-bis(2,6-diisopropylphenyl)-4,5-dihydro-1H-imidazol-3-ium (carbene C), 1,3-bis(2,4,6-trimethylphenyl)-1H-imidazol-3-ium (carbene B), 1,3-bis(2,4,6-trimethylphenyl)-4,5-dihydro-1H-imidazol-3-ium (carbene D), 4,5-dichloro-1,3-bis(2,6-diisopropylphenyl)-1H-imidazol-3-ium (carbene E), 1,3-di-tert-butyl-1H-imidazol-3-ium (carbene F), 1,3-di-tert-butyl-4,5-dihydro-1H-imidazol-3-ium, said salts being in the form of chloride salts.

**7.** Method according to claim 5, **characterised in that** the metallic catalyst is chosen from the salts or complexes of:

- metals chosen from boron, silicon, aluminium, gallium, tin, indium;
- alkaline metals chosen from sodium, potassium;
- alkaline earth metals chosen from magnesium, calcium;
- transition metals chosen from nickel, iron, cobalt, zinc, copper, rhodium, ruthenium, platinum, palladium, iridium;
- rare earths chosen from lanthanum, cerium, praseodymium, neodymium.

**8.** Method according to any one of claims 1 to 7, **characterised in that** the reaction is performed at a $CO_2$ pressure, between 1 and 50 bar, inclusive.

**9.** Method according to any one of claims 1 to 8, **characterised in that** the reaction is performed at a temperature between 25 and 150°C, inclusive.

**10.** Method according to any one of claims 1 to 9, **characterised in that** the reaction is performed for a period of 5 minutes to 72 hours, inclusive.

**11.** Method according to any one of claims 1 to 10, **characterised in that** the reaction is performed in a or in a mixture of at least two solvent (s) chosen from:

- ethers chosen from diethyl ether, or THF;
- hydrocarbons chosen from benzene, or toluene;
- nitrogenous solvents chosen from pyridine, or acetonitrile;
- sulphoxides chosen from dimethyl sulphoxide;
- alkyl halides chosen from chloroform, or methylene chloride.

**12.** Method according to any one of claims 1 to 11, **characterised in that** the molar ratio between the silane compound of formula (III) and the amine of formula (II) is from 1 to 10, preferably from 1 to 3, inclusive.

**13.** Method according to any one of claims 1 to 12, **characterised in that** the quantity of catalyst is from 0.001 to 1 molar equivalent, inclusive, with respect to the amine of formula (II).

**14.** Use of a method for preparing formamide compounds having formula (I) according to any one of claims 1 to 13, in the manufacture of vitamins, pharmaceutical products, adhesives, acrylic fibres, synthetic leathers, pesticides, herbicides, antifungal agents, and fertilisers.

**15.** Method for manufacturing tracers and radioactive tracers, **characterised in that** it comprises a step for preparing nitrogenous compounds of formula (I) using the method according to any one of claims 1 to 13.

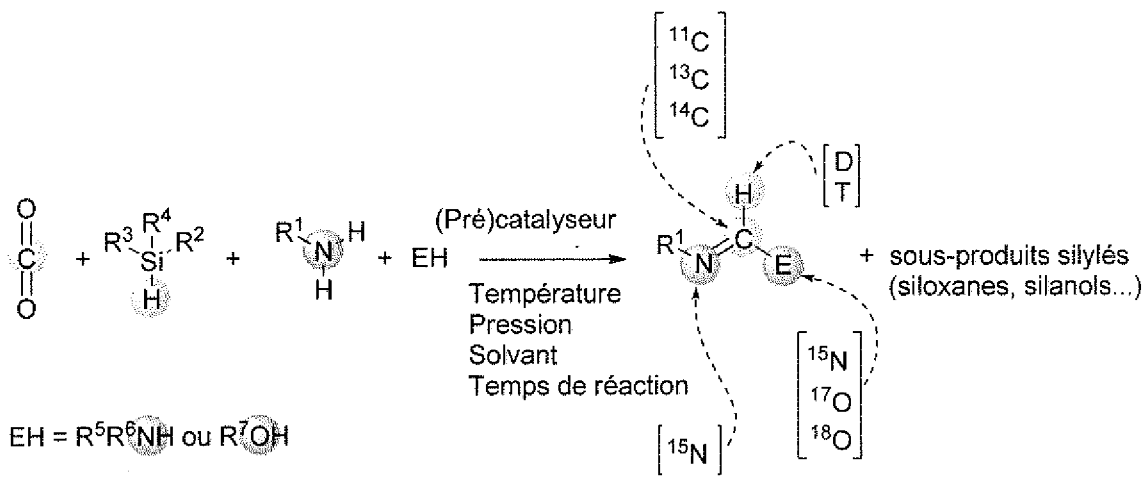
(Pré)catalyseur
Température
Pression
Solvant
Temps de réaction
+ sous-produits silylés
(siloxanes, silanols...)
EH = R5R6NH ou R7OH

**Figure 1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **ALINEZHAD ; HESHMATOLLAT et al.** *Synthetic Communications,* 2012, vol. 42 (1), 102-108
- **WEITH W.** *Chemische Berichte,* vol. 9, 454-458
- **SAKAKURA, T. ; CHOI, J. C. ; YASUDA, H.** *Chem Rev,* 2007, vol. 107, 2365
- **A. J. MORRIS ; G. J. MEYER ; E. FUJITA.** *Acc. Chem. Res.,* 2009, vol. 42, 1983
- **A. BERKEFELD ; W. E. PIERS ; M. PARVEZ.** *J. Am. Chem. Soc.,* 2010, vol. 132, 10660
- **M. GALL ; J. M. MCCALL ; R. E. TENBRINK ; P. F. VONVOIGTLANDER ; J. S. MOHRLAND.** *Journal of Medicinal Chemistry,* 1988, vol. 31, 1816-1820
- **S. ENTHALER ; K. SCHRÔDER ; S. INOUE ; B. ECKHARDT ; K. JUNGE ; M. BELLER ; M. DRIEß.** *European Journal of Organic Chemistry,* 2010, 4893-4901
- **K.-M. CHENG ; Y.-Y. HUANG ; J.-J. HUANG ; K. KANEKO ; M. KIMURA ; H. TAKAYAMA ; S.-H. JUANG ; F. F. WONG.** *Bioorganic and Medicinal Chemistry Letters,* 2010, vol. 20, 6781-6784
- **A. MEKHALFIA ; R. MUTTER ; W. HEAL ; B. CHEN.** *Tetrahedron,* 2006, vol. 62, 5617-5625
- **KHAKSAR, SAMAD ; VAHDAT, SEYED MOHAMMAD ; HEYDARI, AKBAR ; TAJBAKHSH, MAHMOOD.** *Journal of Fluorine Chemistry,* 2010, vol. 131, 1377-1381
- **C.L. SMITH et al.** *Journal of Organometallic Chemistry,* 1974, vol. 81, 33-40
- **G.D. HOMER.** *Journal of the American Chemical Society,* 1973, vol. 95, 23
- **L. SPIALTER et al.** *Journal of the American Chemical Society,* 1971, vol. 93 (22), 5682-5686
- **R. WEST.** *Journal of the American Chemical Society,* 1954, 6015-6017
- **U. PLEISS ; R. VOGES.** Synthesis and Applications of Isotopically Labelled Compounds. Wiley-VCH, 2001, vol. 7
- **R. VOGES ; J. R. HEYS ; T. MOENIUS.** Préparation of Compounds Labeled with Tritium and Carbon-14. Wiley-VCH, 2009
- **YONG-JOO KIM ; MAX P. BERNSTEIN ; ANGELA S. GALIANO ROTH ; FLOYD E. ROMESBERG ; PAUL G. WILLIARD ; DAVID J. FULLER ; AIDAN T. HARRISON ; DAVID B. COLLUM.** *J. Org. Chem.,* 1991, vol. 56, 4435-4439
- **R. VOGES ; J. R. HEYS ; T. MOENIUS.** Preparation of Compounds Labeled with Tritium and Carbon-14. Wiley-VCH, 2009
- **CHRISTOPHER J. DINSMORE ; SWATI P. MERCER.** *Organic Letters,* 2004, vol. 6 (17), 2885-2888
- **JOHN T. GROVES ; YOSHIHITO WATANABE.** *J. Am. Chem. Soc.,* 1988, vol. 110, 8443-8452
- **JOHN T. GROVES ; YOSHIHITO WATANABET.** *J. Am. Chem. SOC.,* 1988, vol. 110, 8443-8452
- **T. A. KOCHINA ; D. V. VRAZHNOV ; E. N. SINOTOVA ; V. V. AVRORIN ; M. YU. KATSAP ; YU. V. MYKHOV.** *Russian Journal of General Chemistry,* 2002, vol. 72 (8), 1222-1224
- **E. A. SHISHIGIN ; V. V. AVRORIN ; T. A. KOCHINA ; E. N. SINOTOVA.** *Russian Journal of General Chemistry,* 2005, vol. 75 (1), 152
- **J. Z. HO.** *Helvetica Chimica Acta,* 2005, vol. 88, 1040
- **I. V. EKHATO ; S. BONACORSI JR., J.** *Label Compd. Radiopharm,* 2011, vol. 54, 202-205
- **KENNETH K. CHAN ; JAMES A. STAROSCIK.** *Journal of Medicinal Chemistry,* 1977, vol. 20 (4), 598